(19) Europäisches Patentamt
European Patent Office
Office européen des brevets

(11) EP 2 185 543 B1

(12) **FASCICULE DE BREVET EUROPEEN**

(45) Date de publication et mention de la délivrance du brevet:
**21.05.2014 Bulletin 2014/21**

(21) Numéro de dépôt: **08828432.8**

(22) Date de dépôt: **29.08.2008**

(51) Int Cl.:
***C07D 401/12*** (2006.01) ***A61K 31/4439*** (2006.01)
***A61P 1/04*** (2006.01) ***C07D 235/28*** (2006.01)
***C07D 213/68*** (2006.01)

(86) Numéro de dépôt international:
**PCT/FR2008/051547**

(87) Numéro de publication internationale:
**WO 2009/027614 (05.03.2009 Gazette 2009/10)**

# (54) PROCEDE DE DEDOUBLEMENT OPTIQUE DE SELS DE L'OMEPRAZOLE

VERFAHREN ZUR OPTISCHEN TRENNUNG VON OMEPRAZOL-SALZEN

PROCESS FOR THE OPTICAL RESOLUTION OF OMEPRAZOLE SALTS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **29.08.2007 FR 0706058**

(43) Date de publication de la demande:
**19.05.2010 Bulletin 2010/20**

(73) Titulaire: **UNIVERSITE DE ROUEN**
**76821 Mont-Saint-Aignan Cédex (FR)**

(72) Inventeurs:
- **COQUEREL, Gérard**
**F-76960 Notre Dame de Bondeville (FR)**
- **TAUVEL, Guillaume**
**F-76000 Rouen (FR)**
- **PETIT, Marie-Noelle**
**F-76130 Mont Saint Aignan (FR)**

(74) Mandataire: **Cabinet Plasseraud**
**52, rue de la Victoire**
**75440 Paris Cedex 09 (FR)**

(56) Documents cités:
**WO-A-98/54171**
**WO-A-2004/016569**
**WO-A-2004/063188**
**WO-A-2006/003163**
**WO-A-2006/131338**
**WO-A1-2005/105786**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 185 543 B1

Printed by Jouve, 75001 PARIS (FR)

## Description

La présente invention concerne le domaine du dédoublement de composés chiraux existant sous la forme de deux antipodes optiques (énantiomères), comme l'Oméprazole.

L'invention permet, à partir du mélange racémique, de séparer l'énantiomère pur (S) (-) Oméprazole (Esoméprazole), de nomenclature chimique 5-méthoxy-2-[(S)-[4-méthoxy-3,5-diméthyl-2-pyridinyl)méthyl]sulfinyl] -1H-benzimidazole et ses sels alcalins pharmaceutiquement acceptables.

Tout spécialement, la présente invention concerne le dédoublement des sels de potassium de l'Oméprazole racémique par cristallisation préférentielle et notamment par le procédé AS3PC (cristallisation préférentielle polythermique programmée et auto-ensemencée).

L'Oméprazole racémique est représenté par la formule générale (I) ci-dessous :

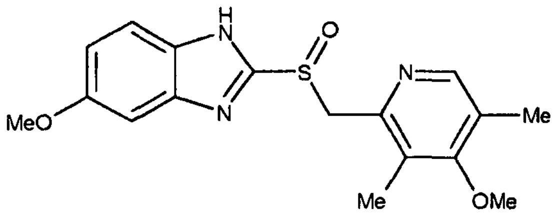

(I)

L'invention se rapporte tant à l'Oméprazole de formule (I) ci-dessus qu'à sa forme tautomère, de même en ce qui concerne ses sels et énantiomères ci-après.

L'énantiomère pur (S) Oméprazole (Esoméprazole) est représenté par la formule générale (II) ci-dessous :

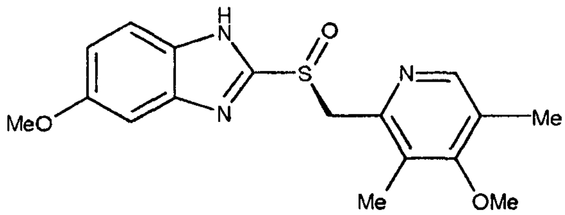

(II)

Cet énantiomère pur est commercialisé en tant que médicament sous forme du sel de magnésium (S) Oméprazole trihydrate d'appellation Nexium®, représenté par la formule générale (III) ci-dessous :

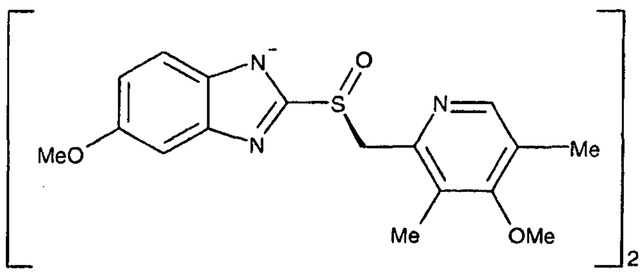

$Mg^{2+}$ $3H_20$

(III)

On peut rappeler que le sel de magnésium de l'énantiomère (S) de l'Oméprazole est le premier inhibiteur de la pompe à protons (IPP) développé et commercialisé sous forme d'énantiomère pur. L'Oméprazole racémique et l'Esoméprazole sont employés comme anti-ulcéreux gastriques et/ou duodénaux. Ils peuvent être utilisés dans la prévention et le traitement de désordres gastro-intestinaux, de reflux gastro-oesophagien, des hémorragies digestives et des dyspepsies. De plus, le (S) Oméprazole peut être utile dans le traitement du psoriasis et dans le traitement des infections à la bactérie *Helicobacter pylori* et des pathologies en relation.

L'Oméprazole et ses énantiomères appartiennent à la classe chimique des prazoles comprenant un noyau benzimidazole ou imidazo-pyridine. Parmi les nombreux prazoles commercialisés ou en cours de développement clinique, peuvent êtres cités : l'Ilaprazole, le Lansoprazole, le Leminoprazole, le Pantoprazole et le Rabéprazole/Pariprazole sous leur forme racémique et/ou énantiomère pur.

Ces prazoles et, plus particulièrement, leurs sels alcalins ou alcalino-terreux correspondants sont utilisés comme inhibiteurs de la sécrétion gastrique acide et comme tels dans les traitements.
Ce sont des sulfoxydes chiraux dont l'atome de soufre lié, d'une part, à un atome d'oxygène et, d'autre part, à des substituants cycles hétéroaromatiques A et méthylène-cycle hétéroaromatiques B différents, constitue le centre stéréogénique.
Ils peuvent être représentés par la formule générale IV ci-dessous :

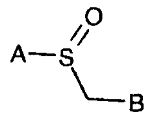
(IV)

Le passage de l'Oméprazole (composé racémique) à l'Esoméprazole (énantiomère pur *lévogyre* (-) de configuration absolue (S)), a constitué, pour cette classe de composés pharmaceutiques IPP, le premier « chiral switching ». Il a été montré que ce composé sous sa forme racémique et chacun de ses énantiomères peuvent présenter des propriétés pharmacologiques et pharmacocinétiques différentes.
L'Oméprazole (composé racémique) a été décrit pour la première fois dans le brevet EP 0 005129 et certains de ses sels alcalins dans les brevets EP 124495 et US 4,738,974.
Les sels alcalins et alcalino-terreux et plus particulièrement le sel de magnésium de l'Oméprazole, et ceux d'autres prazoles IPP, ont montré être stables et pour certains d'entre eux non hygroscopiques.
Un grand nombre de documents de l'art antérieur décrit la préparation de l'Esoméprazole et de ses sels alcalins et alcalino-terreux.
D'une manière générale, ces documents décrivent des procédés classiques qui peuvent être classés selon les procédés mis en jeu :

> Erlandsson P. *et coll.* ont publié le premier dédoublement de l'Oméprazole racémique réalisé par chromatographie sur phase chirale dans J. Chromatogr., 1990, 532, 305-319.

Les demandes de brevet DE 40 35 455 et WO 94/27988 décrivent le dédoublement de l'Oméprazole racémique et de prazoles similaires de type pyridinylméthylsulfinyl-1H-benzimidazoles par cristallisation fractionnée de sels diastéréomères et/ou séparation chromatographique en phase inverse d'éthers diastéréomères covalents de type N-acyloxyméthyl chiraux liés à l'atome d'azote N libre du cycle benzimidazole, suivies d'hydrolyse basique.
La demande de brevet US 2006/0089386 décrit le dédoublement de l'Oméprazole racémique à l'aide du (S) chlorure de camphorsulfonyle par formation de diastéréomères covalents séparés par cristallisation fractionnée suivie d'hydrolyse basique pour donner le (S) Oméprazole > 99% ee.
La demande de brevet WO 96/01623 décrit la formation des sels de magnésium des énantiomères (R) et (S) Oméprazole d'excès énantiomérique 99% ee et de forme cristalline I.
La demande de brevet WO 96/17077 décrit un procédé de bioréduction stéréosélective du sulfoxyde (Oméprazole racémique) en sulfure (thio-éther) correspondant à l'aide de micro-organismes tels que *Escherichia coli, Proteus mirabilis* ou *Proteus vulgaris,* contenant l'enzyme DMSO réductase ou à l'aide de cet enzyme purifié. Cette bioréduction énantiosélective laisse l'Oméprazole fortement enrichi en énantiomère (+) avec un excès énantiomérique 99% ee. L'énantiomère (-) est obtenu avec 70% ee.
La demande de brevet WO 96/17076 décrit un procédé de biooxydation stéréosélective du sulfure (thio-éther), précurseur de l'Oméprazole, en présence de micro-organismes tels que *Penicillium frequentans, Brevibacterium paraffinolyticum* ou *Mycobactérium sp.,* pour donner l'énantiomère (S) Oméprazole de 99% ee.
La demande de brevet WO 96/02535 décrit la synthèse asymétrique mettant en jeu l'oxydation asymétrique du sulfure pro-chiral à l'aide du système catalytique, $Ti(O\text{-}isoPr)_4$/di-éthyl-D-tartrate/$H_2O$ en présence d'hydroperoxyde de cumène, de base organique amine tertiaire et de solvant organique tel que le toluène, suivie de formation *in situ* de sels de sodium correspondants. Ce procédé catalytique permet d'obtenir des sulfoxydes chiraux, notamment le sel de sodium du (S) Oméprazole de 99% ee.
La demande de brevet WO 2006/040635 décrit la synthèse énantiosélective de pyridinylméthylsulfinylbenzimidazoles par oxydation catalytique des dérivés sulfures prochiraux, précurseurs correspondants, à l'aide du même système catalytique, $Ti(O\text{-}isoPr)_4$/di-éthyl-D-tartrate/$H_2O$, hydroperoxyde de cumène, base organique amine tertiaire sans ajout de solvant organique pour donner l'énantiomère (S) Oméprazole et ses sels alcalins et alcalino-terreux, notamment de magnésium.
La demande de brevet WO 03/089408 décrit la synthèse asymétrique du (S) Oméprazole par oxydation énantiosélective catalytique du sulfure pro-chiral précurseur à l'aide d'un ligand chiral monodenté de type ester méthylique

de l'acide L-mandélique en présence d'hydroperoxyde de cumène, de Ti(O-isoPr)$_4$/$H_2O$ et préparation in situ du sel de sodium correspondant de 99% ee.

La demande de brevet WO 98/28294 décrit la préparation du (S) Oméprazole solide sous une forme amorphe, une forme cristalline (désignée forme A) ou partiellement cristalline (désignée forme B).

La demande de brevet WO 98/54171 décrit la formation des sels de magnésium des énantiomères (S) et (R) de l'Oméprazole dihydrate sous les formes cristallines A et B et du trihydrate. Ce brevet décrit aussi la formation des sels de potassium des énantiomères (S) et (R) de l'Oméprazole.

La demande de brevet WO 00/44744 décrit la formation d'un nouveau sel de potassium du (S) Oméprazole hydrate de forme B.

La demande de brevet WO 2004/002982 décrit la séparation de l'Oméprazole racémique en ses énantiomères purs par formation de sels diastéréomères à partir du sel de sodium de l'Oméprazole racémique mis en présence de l'agent de coordination diéthyl-D-Tartrate/Ti(iso-Pr)$_4$ dans l'acétone et complexation à l'aide de l'acide L-mandélique.

La cristallisation sélective du diastéréomère comprenant le (S) Oméprazole suivie d'une hydrolyse basique donne le sel de magnésium (S) Oméprazole trihydrate de 99% ee. La formation du dihydrate correspondant est obtenue par séchage contrôlé.

La demande de brevet WO 2004/046134 décrit la préparation du sel de magnésium du (S) Oméprazole trihydrate de forme cristalline II à partir de la forme amorphe du même sel.

La demande de brevet WO 97/02261 (EP 1498416) décrit l'enrichissement énantiomérique de mélanges d'énantiomères (S)/(R) d'Oméprazole par précipitation sélective du composé racémique correspondant dans un solvant de type acétone ou acétonitrile. Le filtrat après évaporation, donne le (S) Oméprazole de 98-99% ee.

Dans J. Phys. IV, 2004, 113, 11-15, Coquerel G. présente le rationnel supportant la formation de l'anti-conglomérat (correspondant à l'Oméprazole racémique) et sa cristallisation à l'aide des diagrammes de phases binaires (voir Fig. 5b de cette publication).

La demande de brevet WO 2004/089935 décrit la préparation d'une nouvelle forme cristalline du sel de magnésium du (S) Oméprazole trihydrate, dénommée HI et l'accès au sel de magnésium du (S) Oméprazole hémihydrate et du (S) Oméprazole monohydrate, chacun caractérisé par des diagrammes de diffraction X sur poudre.

La demande de brevet WO 2006/001753 décrit la préparation du (S) Oméprazole et des sels de sodium obtenus sous des formes cristallines identifiées C, E et H à partir du sel de potassium précurseur correspondant traité en milieu basique.

La demande de brevet WO 2006/003163 décrit la préparation de nouvelles formes cristallines de (S) Oméprazole solvate de méthanol, caractérisées par des diagrammes de diffraction X sur poudre.

La demande de brevet WO 2006/134605 décrit la formation du (S) Oméprazole hydrate amorphe, et sa transformation en anhydre par remise en suspension dans un solvant organique et filtration.

La demande de brevet WO 2004/076440 décrit les formes I et II du (S) Oméprazole et ses hydrates.

La demande de brevet WO 2004/020436 décrit des hydrates amorphes du sel de magnésium du (S) Oméprazole et leur préparation.

La demande de brevet WO 2007/031845 décrit la préparation du sel de magnésium (S) Oméprazole trihydrate sous deux formes cristallines nouvelles polymorphes G1 et G2 ainsi que la préparation d'une forme amorphe correspondante.

La demande de brevet WO 2007/049914 décrit la formation d'un sel de strontium du (S) Oméprazole tétrahydrate sous une forme cristalline A et une forme amorphe.

Dans Tetrahedron : Asymmetry, 2000, 11, 1729-1732, Deng J. *et coll.* décrivent le dédoublement de l'Oméprazole racémique par formation de complexes d'inclusion à l'aide du (S)-(-)-2,2'-dihydroxy-1-1'-binaphthyl (BINOL), suivie de cristallisation et de séparation chromatographique pour donner le (S) Oméprazole de 99% ee.

La demande de brevet WO2007/074099 décrit le dédoublement de l'Oméprazole racémique par formation de complexes d'inclusion à l'aide du ligand chiral (S)-1,1,6-triphényl-1,2-éthanediol. Du complexe formé de (S) Oméprazole et 2 équivalents de ligand chiral est récupéré le (S) Oméprazole cristallisé de 99% ee.

Si certains de ces procédés sont utilisés sur le plan industriel, l'évolution de la réglementation, notamment à propos de la sécurité et de la qualité des produits pharmaceutiques ainsi que l'impact économique des décisions des organismes officiels de santé vers un meilleur contrôle des coûts des traitements, imposent le développement et l'optimisation de nouvelles méthodes de préparation de l'énantiomère (S) Oméprazole et de ses sels d'intérêt pharmaceutique.

Parmi les procédés existants décrits dans les brevets ci-dessus, on peut citer, de manière non exhaustive, un ensemble d'inconvénients couramment rencontrés et directement liés à l'utilisation de ces procédés. Plusieurs de ces inconvénients pouvant se trouver dans un seul et même procédé :

- Dans le cas de la synthèse asymétrique catalytique mettant en jeu des catalyseurs de type Ti(O-isoPr)$_4$, trioxyde de vanadium ou d'acétylacétonate de tungstène, la présence de métaux lourds résiduels.

- La formation de la sulfone correspondant à l'oxydation complète de l'atome de soufre, pouvant être obtenue jusqu'à 40% par rapport au (S) Oméprazole et difficilement séparable par chromatographie ou recristallisation.
- La nécessité de passer par une étape de séparation chromatographique et de purification énantiomérique par recristallisation quand les méthodes de synthèse asymétrique fournissent le (S) Oméprazole de pureté chimique < 90% et de pureté énantiomérique < 95% ee.
- L'utilisation d'une méthode de séparation/purification chromatographique HPLC sur phase chirale pour les méthodes chromatographiques directes sans mise en jeu de diastéréomères covalents ou sels.
- En cas de pureté structurale insuffisante (Coquerel G., The 'structural purity' of molecular solids
- An elusive concept ? Chem. Eng. Process 2006, 45, 857-862.) quand l'énantiomère obtenu sous sa forme neutre ou sous forme de sel alcalin ou alcalino-terreux, notamment de sel de magnésium, est un mélange de formes, d'hydrates et/ou de solvates de stabilités différentes.
- L'élimination de micro-organismes et d'enzymes résiduels dans le cas des biotransformations.

La présente invention a précisément pour but d'offrir une méthode de préparation de l'énantiomère pur (S) Oméprazole qui ne présente pas les inconvénients décrits ci-dessus.

Ce but est atteint grâce à l'application du procédé de cristallisation préférentielle à l'Oméprazole racémique sous forme de sel. Ainsi, l'invention se rapporte tout particulièrement à l'application à des sels de potassium de l'Oméprazole racémique, du dédoublement par cristallisation préférentielle en chacun de ses énantiomères, permettant d'obtenir l'eutomère (S) Oméprazole dans une forme énantiomériquement et chimiquement pure.

En particulier, le procédé de cristallisation préférentielle AS3PC a fait l'objet d'un développement tout à fait original excluant l'utilisation contraignante de germes de cristallisation. Ce procédé est décrit par exemple dans les brevets et demandes de brevet suivants FR 2 710 337, WO 95/08522, EP 0 720 595 et US 6,022,409 et dans « G. Coquerel, Preferential Crystallization in Topic in Current Chemistry, Novel Optical Resolution Technol ogi es, Springer, Berlin- Hei delberg, Eds K. Sakai, N. Hirayama and R. Tamura », 2007, 269, 1-51. Ce procédé est désigné « AS3PC », pour "Auto-Seeded Programmed Polythermic Preferential Crystallization".

Les procédés de cristallisation préférentielle reposent sur la cristallisation alternée des deux énantiomères (R) et (S), d'une même espèce chimique racémique cristallisant sous forme de conglomérat, dans un médium qui peut être un solvant ou un mélange de solvants ou un ensemble de constituants incluant le ou les solvants, et ce pour une gamme donnée de température $\Delta T$. Dans cette gamme de température, ce mélange racémique, en équilibre thermodynamique avec sa solution saturée, est constitué de deux types de cristaux ne contenant chacun que des molécules de même configuration absolue. Chaque énantiomère peut incorporer, des molécules de solvant (solvates) et/ou d'eau (hydrates).

La connaissance de ces équilibres hétérogènes énantiomère (R) - énantiomère (S) - médium fournit des données mises à profit pour réaliser un dédoublement efficace par cristallisation préférentielle.

Les études menées par la Demanderesse montrent que l'Oméprazole racémique n'est pas un conglomérat. Cela signifie que l'on ne peut pas appliquer le procédé de cristallisation préférentielle AS3PC ou tout autre procédé de cristallisation préférentielle. Il en est de même pour les sels de sodium et de magnésium.

Par contre, de manière tout à fait inattendue, la Demanderesse a trouvé, que les sels de potassium de l'Oméprazole racémique sous forme de solvates d'éthanol ou d'éthylèneglycol sont des conglomérats sans solution solide détectable. Le domaine éventuel de miscibilité à l'état solide serait inférieur à 1%, comme illustré à la figure 1.

Ainsi, de façon surprenante, les sels de potassium de l'Oméprazole racémique, d'un mélange enrichi en énantiomère (S) de l'Oméprazole ou de l'énantiomère pur (S) Oméprazole sont stables en présence d'un excès de potasse dans les mélanges de solvants alcool-alcool, alcool-eau ou alcool pur. Dans ces conditions, de concentration et de température, ces sels de potassium présentent une solubilité non-congruente (G. Coquerel dans « Preferential Crystallization, in Topic in Current Chemistry, Novel Optical Resolution Technologies, Springer, Berlin- Heidelberg, Eds K. Sakai, N. Hirayama and R. Tamura », 2007, 269, 1-51). Cela signifie que pour obtenir de manière quantitative ce sel d'excès énantiomérique supérieur à 99% ee il est nécessaire de travailler en milieu alcoolique avec un excès de potasse.

Ainsi, l'invention se rapporte à un procédé de dédoublement de l'Oméprazole racémique, caractérisé en ce que l'on transforme l'Oméprazole racémique en son sel de potassium sous forme de solvate, en présence d'un excès de base minérale source de potassium, ledit sel de potassium de l'Oméprazole racémique sous forme de solvate se présentant sous la forme de conglomérats dont les domaines de solution solide partielle, s'ils existent, sont inférieurs à 1%, puis en ce que l'on dédouble lesdits conglomérats par cristallisation préférentielle pour séparer les deux énantiomères (S) et (R) dudit sel de potassium de l'Oméprazole.

Dans le procédé ci-dessus, le solvate du sel de potassium de l'Oméprazole racémique est choisi parmi le solvate d'éthanol ou le solvate d'éthylèneglycol ou un mélange de ceux-ci.

Le dédoublement des conglomérats selon l'invention est réalisé par cristallisation préférentielle ensemencée ou autoensemencée.

Un premier mode de réalisation de l'invention est un procédé de dédoublement par cristallisation préférentielle non autoensemencée (i.e. ensemencée) d'un sel de l'Oméprazole racémique ; ce procédé comprend les étapes

suivantes :

a) on prépare une première solution homogène composée du mélange racémique sous forme de conglomérat et d'un excès du premier énantiomère à récupérer sous forme de solvate du sel de potassium du (X) Oméprazole, désigné X-K-solvate, où X représente l'énantiomère (R) ou (S), et d'un médium, dont le point figuratif I (figure 2), défini par les variables concentration et température $T_I$ ($T_I > T_{HOMO}$), se situe dans le domaine monophasé composé de la solution sous saturée ;
b) on applique une loi de programmation de refroidissement au mélange monophasé ;
c) lorsque le mélange atteint une température inférieure à la température $T_{HOMO}$, on ensemence la solution avec des germes énantiomériquement purs du premier énantiomère X-K-solvate à récupérer ;
d) on adapte pendant toute la durée de la croissance cristalline une vitesse d'agitation légèrement croissante en fonction du temps de façon à ce que celle-ci soit suffisamment lente pour favoriser une croissance du premier énantiomère X-K-solvate ;
e) on récolte les cristaux du premier énantiomère X-K-solvate ;
f) on ajoute aux liqueurs mères la même masse de mélange racémique que la masse de la récolte réalisée à l'étape précédente, et on porte le nouvel ensemble à la température $T_I$ ($T_I > T_{HOMO}$), le point I' se situant dans le domaine monophasé (ce point I' correspond au symétrique du point I par rapport au plan de composition équimolaire ou équimassique en les deux énantiomères) ;
g) on applique la même loi de programmation de refroidissement qu'à l'étape (b) au mélange monophasé préparé à l'étape (f) contenant le second énantiomère, de sorte que les liqueurs mères gardent une faible sursaturation pendant la cristallisation afin de privilégier la croissance du second énantiomère X-K-solvate lors de l'ensemencement ;
h) lorsque le mélange atteint une température inférieure à la température $T_{HOMO}$, on ensemence la solution avec des germes énantiomériquement purs du second énantiomère X-K-solvate ;
i) on adapte pendant toute la durée de la croissance cristalline de l'étape précédente, une vitesse d'agitation légèrement croissante en fonction du temps de façon à ce que celle-ci soit, suffisamment lente pour favoriser la croissance de ce second énantiomère X-K-solvate;
j) on récolte les cristaux du second énantiomère X-K-solvate ;
k) on ajoute aux liqueurs mères la même masse de mélange racémique que la masse de la récolte réalisée à l'étape précédente, pour obtenir une solution dont la composition est identique à celle de la solution initiale ;
l) on répète les étapes (a) à (k) pour obtenir successivement l'un puis l'autre des deux énantiomères.

À l'étape (a) du procédé ci-dessus, le médium est constitué par un solvant alcoolique ou mélange de solvants alcooliques, de l'eau et un excès de potasse.

Selon un deuxième mode de réalisation de l'invention, le procédé de dédoublement des conglomérats peut être réalisé par cristallisation préférentielle et tout particulièrement par le procédé AS3PC, « Auto-Seeded Programmed Polythermic Preferential Crystallization ». Ainsi, un procédé de dédoublement AS3PC d'un sel de l'Oméprazole racémique selon l'invention comprend les étapes suivantes :

a) on prépare un premier ensemble composé du mélange racémique sous forme de conglomérat, du premier énantiomère à récupérer, sous forme de solvate du sel de potassium du (X) Oméprazole, désigné X-K-solvate, où X représente l'énantiomère (R) ou (S), et d'un médium, dont le point figuratif E (figure 2bis), défini par les variables concentration et température $T_B$, se situe dans le domaine biphasé composé de cristaux de X-K-solvate et de sa solution saturée (ce point E' correspond au symétrique du point E par rapport au plan de composition équimolaire ou équimassique en les deux énantiomères) ;
b) on applique une loi de programmation de refroidissement au mélange biphasé, telle que les liqueurs mères gardent une faible sursaturation qui privilégie la croissance du premier énantiomère X-K-solvate présent sous forme de cristaux, tout en interdisant la nucléation spontanée du second énantiomère X-K-solvate dissout dans la solution ;
c) on adapte pendant toute la durée de la croissance cristalline une vitesse d'agitation légèrement croissante en fonction du temps de façon à ce que celle-ci soit suffisamment lente pour favoriser une croissance du premier énantiomère X-K-solvate, en évitant de générer une nucléation non maîtrisée et l'attrition de cristaux ;
d) on récolte les cristaux du premier énantiomère X-K-solvate ;
e) on ajoute aux liqueurs mères la même masse de mélange racémique que la masse de la récolte réalisée à l'étape précédente, et on porte le nouvel ensemble à la température $T_B$ le point E' se situant dans le domaine biphasé du second énantiomère X-K-solvate en excès, en équilibre avec sa solution saturée ;
f) on applique la même loi de programmation de refroidissement qu'à l'étape (b) au mélange biphasé préparé à l'étape (e) contenant le second énantiomère X-K-solvate, de sorte que les liqueurs mères gardent une faible sursaturation pendant la cristallisation afin de privilégier la croissance du second énantiomère X-K-solvate présent

sous forme de cristaux tout en interdisant la nucléation spontanée du premier énantiomère X-K-solvate présent dans la solution ;

g) on adapte pendant toute la durée de la croissance cristalline de l'étape précédente, une vitesse d'agitation légèrement croissante en fonction du temps de façon à ce que celle-ci soit, suffisamment lente pour favoriser la croissance de ce second énantiomère X-K-solvate en évitant de générer une nucléation non maîtrisée et l'attrition de cristaux ;

h) on récolte les cristaux du second énantiomère X-K-solvate ;

i) on ajoute aux liqueurs mères la même masse de mélange racémique que la masse de la récolte réalisée à l'étape précédente, pour obtenir un ensemble dont la composition est identique à celle de l'ensemble E initial ;

j) on répète les étapes (a) à (i) pour obtenir successivement l'un puis l'autre des deux énantiomères.

À l'étape (a) du procédé ci-dessus, le médium est constitué par un solvant alcoolique ou mélange de solvants alcooliques, de l'eau et un excès de potasse.

Le procédé de l'invention dans ses diverses formes de mise en oeuvre est simple, économique et aisé ; il ne nécessite pas l'utilisation d'intermédiaires chiraux de type organique, organométallique et/ou d'agents de dédoublement utilisés sous formes de sels ou de diastéréomères covalents ou de micro-organismes appropriés.

Dans le médium, le solvant ou mélange de solvants est de type alcoolique et de préférence choisi parmi l'éthanol, l'éthylèneglycol, pur ou en présence d'eau avec un excès de potasse.

Avantageusement dans le procédé de l'invention, le mélange racémique, mélange équimolaire des énantiomères, dans le médium utilisé, est pour l'intervalle de température $T_B$-$T_F$ ou $T_{HOMO}$-$T_F$, un conglomérat.

Selon une caractéristique utile, le mélange à dédoubler est stable dans ce médium et dans la gamme de température utilisée entre $T_B$ et $T_F$ ou $T_{HOMO}$-$T_F$.

Dans le procédé de l'invention, la température $T_L$ correspond à la température de dissolution du mélange racémique seul, la température $T_{HOMO}$ correspond à la température d'homogénéisation de la solution enrichie en un des énantiomères, la température $T_I$ correspond à la température initiale du procédé de cristallisation préférentielle ensemencé telle que $T_I > T_{HOMO}$ et la température $T_B$ correspond à la température initiale du procédé de cristallisation préférentielle AS3PC telle que $T_L < T_B < T_{HOMO}$. La connaissance des capacités de sursaturation des solutions entre $T_L$ et $T_F$ est également utile, suivant la cinétique de refroidissement. Le temps d'apparition des cristaux par nucléation primaire dans la solution racémique L (cf. figures 1, 2, 2bis et 3) homogène, refroidie à partir d'une température légèrement supérieure à $T_L$ avec la même cinétique, donne une indication sur la capacité de sursaturation tolérée par le conglomérat dans ces conditions expérimentales.

Dans le procédé de l'invention, il est aussi bien de connaître la cinétique de dissolution d'une masse connue de mélange racémique (de granulométrie donnée) dispersée dans la solution à la température $T_B$.

Dans le procédé de l'invention, les systèmes mis en oeuvre (étape (a) sont choisis parmi :

- des systèmes quinaires [$K_2O$ - EtOH - $H_2O$ - (-) Oméprazole - (+) Oméprazole] et [$K_2O$ - Ethylèneglycol-$H_2O$ - (-) Oméprazole - (+) Oméprazole] ;
- un système sénaire [$K_2O$ - EtOH - Ethylèneglycol - $H_2O$ - (-) Oméprazole - (+) Oméprazole] ;
- des systèmes ternaires, par assimilation des systèmes quinaires à des systèmes ternaires comprenant les constituants : alcool(s) (éthanol et/ou éthylèneglycol), eau et excès de potasse dans le médium.

Pour le système sénaire, cette simplification n'est pas possible, mais on pourra considérer des systèmes ternaires juxtaposés donnant lieu à une co-cristallisation préférentielle.

La détermination des différentes formes cristallines ou amorphes est obtenue à l'aide d'une méthode analytique telle que la diffraction des rayons X sur poudre (XRPD). Cette méthode bien connue de l'homme de l'art est capable de produire une analyse qualitative des formes éventuellement présentes dans un même échantillon solide, excepté pour les phases amorphes.

Le dédoublement par cristallisation préférentielle a été étudié de manière systématique dans l'éthanol et dans des mélanges éthanol/eau et éthanol/éthylèneglycol en utilisant le procédé AS3PC tel qu'il a été rappelé ci-dessus et décrit en détail dans WO 95/08522. Il a été également étudié dans le mélange azéotropique éthanol/eau en utilisant le procédé ensemencé.

Chaque sel de potassium de l'Oméprazole solvaté par l'éthanol ou l'éthylèneglycol a été caractérisé par diffraction des rayons X sur poudre. Les diffractogrammes (XRPD) de ces phases racémiques et énantiomériquement pures sont présentés respectivement sur les figures 4 et 5.

De plus, le sel de potassium de l'Oméprazole solvate d'éthanol a été caractérisé par diffraction des rayons X sur monocristal.

Le tableau I ci-dessous montre la position et l'intensité relative des pics caractéristiques pour le solvate d'éthanol du sel de potassium de l'Oméprazole racémique et pour le solvate d'éthanol du sel de potassium du (S) Oméprazole

énantiomériquement pur.

Tableau I

| Solvate d'éthanol du sel de potassium de l'Oméprazole racémique | | Solvate d'éthanol du sel de potassium du (S) Oméprazole | |
|---|---|---|---|
| Angle 2-Theta (2θ) | Intensité I/Io % | Angle 2-Theta (2θ) | Intensité I/Io% |
| 6.42 | 100 | 6.37 | 100 |
| 11.22 | 5 | 11.17 | 10.4 |
| 14.11 | 6.5 | 14.07 | 14.2 |
| 14.89 | 21.4 | 14.88 | 24.3 |
| 16.12 | 9.3 | 16.04 | 15.6 |
| 16.41 | 8 | 16.34 | 16.3 |
| 18.12 | 10.5 | 18.09 | 21.5 |
| 18.93 | 5.2 | 18.87 | 16.5 |
| 20.09 | 9 | 20.08 | 17.7 |
| 22.24 | 4.1 | 22.19 | 12.9 |
| 23.11 | 11 | 23.06 | 15.3 |
| 24.05 | 14 | 23.99 | 26.1 |
| 24.53 | 11.9 | 24.51 | 17.6 |
| 25.20 | 6.2 | 25.16 | 15.4 |
| 25.86 | 5.9 | 25.8 | 16 |
| 27.14 | 12.5 | 27.06 | 17.9 |

Il est à noter qu'après plusieurs semaines le solvate d'éthanol du sel de potassium de l'Oméprazole sous atmosphère ambiante tend à se transformer en sel de potassium du (S) Oméprazole dihydrate.

Cependant, ces solvates d'éthanol du sel de potassium de l'Oméprazole racémique et du (S) Oméprazole sont stables dans un mélange éthanol - eau, majoritaire en éthanol et avec un excès de potasse.

Le tableau II ci-dessous montre la position et l'intensité relative des pics caractéristiques pour le solvate d'éthylèneglycol du sel de potassium de l'Oméprazole racémique et pour le solvate d'éthylèneglycol du sel de potassium du (S) Oméprazole énantiomériquement pur.

Tableau II

| Solvate d'éthylèneglycol du sel de potassium de l'Oméprazole racémique | | Solvate d'éthylèneglycol du sel de potassium du (S) Oméprazole | |
|---|---|---|---|
| Angle 2-Theta (2θ) | Intensité I/Io % | Angle 2-Theta (2θ) | Intensité I/Io % |
| 6.67 | 100 | 6.63 | 100 |
| 10.99 | 4.7 | 10.96 | 1.7 |
| / | / | 12.99 | 0.7 |
| / | / | 14.52 | 1.3 |
| 15.75 | 5.1 | 15.67 | 3.2 |
| 16.04 | 6.1 | 16.03 | 1.4 |
| 17.98 | 17 | 18.03 | 1.3 |
| 20.20 | 6.1 | 20.20 | 4.2 |
| 22.07 | 5.5 | 21.92 | 1.2 |

(suite)

| Solvate d'éthylèneglycol du sel de potassium de l'Oméprazole racémique | | Solvate d'éthylèneglycol du sel de potassium du (S) Oméprazole | |
|---|---|---|---|
| Angle 2-Theta (2θ) | Intensité I/Io % | Angle 2-Theta (2θ) | Intensité I/Io % |
| / | / | 22.41 | 1.1 |
| 24.08 | 5.1 | 24.09 | 3.1 |
| / | / | 24.33 | 1.4 |
| / | / | 24.60 | 2.7 |
| 24.80 | 7.4 | 24.84 | 2.2 |
| / | / | 25.07 | 1.7 |
| / | / | 26.17 | 1.1 |
| / | / | 26.95 | 2.4 |
| 28.49 | 5.7 | 28.55 | 0.9 |
| 29.28 | 4.1 | 29.27 | 0.9 |

Le procédé de l'invention conduisant aussi à l'obtention de l'énantiomère (R), celui-ci peut être recyclé suivant les étapes suivantes :

i) Réduction de ce sulfoxyde chiral (R) en sulfure (thio-éther) achiral. Cette réduction s'opère avantageusement selon les conditions opératoires douces décrites dans la revue de Madesclaire M, Tetrahedron, 1988, 44, 6537-6580, telles que la réaction d'Allenmark S., Acta. Chem. Scand., 1966, 20, 910-911 ou dans WO 2004/016569,
ii) Oxydation de l'atome de soufre de ce sulfure en sulfoxyde racémique, à l'aide d'eau oxygénée ou d'ion hypochlorite ou d'acide perbenzoïque ou tout autre oxydant en évitant la formation de sulfone.

L'Oméprazole racémique ainsi obtenu peut alors être soumis à la cristallisation préférentielle comme décrit ci-dessus.
D'autres avantages et caractéristiques de l'invention apparaîtront des exemples qui suivent et dans lesquels il sera fait référence aux figures ci-jointes où :

- La figure 1 est une représentation en perspective d'une partie du système ternaire polythermique : médium - énantiomère (R) - énantiomère (S), ainsi que des nappes de cristallisation de chaque constituant et des compositions des solutions doublement saturées (courbes monovariantes).

Sur cette figure la coupe verticale isoplèthe contenant le segment S-K-solvate et la composition $X_E$ est représentée.

- La figure 2 est la coupe verticale isoplèthe polythermique extraite de la figure 1 (avec les mêmes symboles). Elle détaille le cheminement du point solution (en trait gras) de I à F lors du dédoublement par cristallisation préférentielle ensemencée.
- La figure 2bis est la coupe verticale isoplèthe polythermique extraite de la figure 1 (avec les mêmes symboles). Elle détaille le cheminement du point solution (en trait gras) de B à F lors du dédoublement par le procédé autoensemencé : AS3PC.
- La figure 3 est une projection sur le plan des concentrations du cheminement du point solution (en trait gras) lors du dédoublement par le procédé de l'invention AS3PC. La coupe polythermique S-K-solvate - Y de la figure 2 est représentée par le segment S-K-solvate - Y.
- La figure 4 montre les diffractogrammes du solvate d'éthanol du sel de potassium du (S) Oméprazole calculé et expérimental et celui du solvate d'éthanol du sel de potassium d'Oméprazole racémique.
- La figure 5 montre les diffractogrammes du solvate d'éthylèneglycol du sel de potassium de l'Oméprazole racémique et du (S) Oméprazole.
- La figure 6 montre la représentation 3D de la maille cristalline du solvate d'éthanol du sel de potassium du (S) Oméprazole.

**Méthodes analytiques utilisées**

Détermination de l'excès énantiomérique (% ee)

Les excès énantiomériques sont déterminés par chromatographie HPLC chirale à l'aide d'une colonne Chiral-PAK AD (dimension 250 mm x 4,6 mm). Les conditions expérimentales sont :

- Solvant : 100% Ethanol absolu
- Débit : 1 ml.min$^{-1}$
- Détecteur : $\lambda$ = 302 nm
- Injection : 20 $\mu$L
- Concentration : environ 0,4 g.l$^{-1}$ dans l'éthanol
- Temps de rétention de l'énantiomère (-) : 8,1 minutes.

Analyses par diffraction des RX sur poudre

Les analyses de diffraction des rayons X sur poudre (XRPD) ont été effectuées à l'aide d'un diffractomètre Bruker D5000 Matic dans les conditions suivantes :

- Anticathode cuivre, voltage 40 kV, intensité 40mA
- Température ambiante
- Domaine de mesures : 3° à 30°
- Incrémentation entre chaque mesure : 0,04°
- Temps de mesure par pas : 4 s.

Mesures de solubilité

Les mesures de solubilité sont calculées, pour une température donnée et pour un excès de potasse donné, de la manière suivante:

$$\frac{\left(\dfrac{\text{Masse d'Oméprazole du sel de potassium de l'Oméprazole} \times \text{masse molaire du solvate}}{\text{masse molaire de l'Oméprazole}}\right)}{\text{masse de l'Oméprazole} + \text{masse de potasse} + \text{masse de solvant} + \text{masse d'eau}}$$

**Dispositif expérimental**

Les opérations sont effectuées alternativement dans deux tubes à col rodé (29/32), excepté à l'échelle de 2 litres, c'est-à-dire pour les exemples 5 et 6. Ces tubes mesurent 19 cm de hauteur et de 45 mm de diamètre pour les exemples 1, 2, 3, 4 et 7, et environ 12 cm de hauteur et 29 mm de diamètre pour l'exemple 8. Ces tubes sont munis, dans leur partie supérieure, d'un tube latéral pour établir une dépression nécessaire à la filtration. Les cristaux sont récupérés sur verre fritté n° 2 ou 3, ou sur Büchner, adaptable sur chaque tube par l'intermédiaire d'un anneau de caoutchouc. L'agitation est assurée par un barreau magnétique. Les liqueurs mères passent successivement d'un tube à l'autre.

Ces transferts, réduits au minimum, n'empêchent pas des pertes à chaque opération. Afin de compenser ces pertes, deux actions de compensation sont ainsi conduites :

- pour les pertes de liqueur mère, sur le verre fritté et dans le tube initial, cette compensation se fait par ajout de cristaux racémiques et de solvant, de telle sorte que cette addition corresponde au mélange L,
- pour les pertes en solvant, principalement dues à la filtration créée par dépression, la compensation se fait par ajout à chaque opération de solvant supplémentaire.

Pour un solvant très volatil, le procédé de compensation est affiné. Une petite quantité de la solution est prélevée, afin d'en déterminer la composition, permettant ensuite une compensation rigoureuse.

Afin d'accéder à une bonne reproductibilité des résultats, le liquide caloporteur circulant dans la double enveloppe de chaque chambre de cristallisation est régulé en température avec une précision de ± 0,1° C. L'appareillage mis en oeuvre permet de fixer une loi de refroidissement reproductible. Ces chambres de cristallisation sont thermostatées à l'aide d'un thermostat (LAUDA RE107) muni d'une jaquette à double enveloppe.

Dans les exemples 5 et 6, les opérations sont réalisées dans un réacteur de 2 litres à double enveloppe thermostaté par un thermostat (Huber CC 415) et muni d'une vanne de fond. L'agitation est mécanique et est assurée au moyen d'une pale à double hélice. La filtration a lieu au moyen d'une essoreuse (RA20 Rousselet-Robatel) à 5000 tours/min munie d'une chaussette de 20 cm de diamètre, de 10 cm de hauteur et dont le diamètre des pores du media filtrant en nylon est de 20 μm. La liqueur mère récupérée est transvasée dans le réacteur pour la cristallisation préférentielle suivante.

Il est à noter que les compensations après chaque opération sont effectuées en ajoutant de l'Oméprazole racémique et de la potasse (KOH) ; ce qui revient à décaler en composition le mélange par formation d'eau, résultant de la salification.

**<u>Exemple 1 : Dédoublement dans le mélange azéotropique éthanol/eau par cristallisation préférentielle ensemencée</u>**

<u>Conditions liées aux équilibres :</u>

Solubilité du mélange racémique dans le mélange azéotropique éthanol/eau contenant 1,2 équivalent molaire de potasse (soit un excès de potasse de 0,2 équivalent)

Coordonnées du point L : 9,1% massique ; température $T_L$ : 31°C

| Température (°C) | 29 | 31 | 35 |
|---|---|---|---|
| Solubilité (% massique) | 8,7 | 9,1 | 13,7 |

Évolution de $T_{HOMO}$ avec l'excès énantiomérique :

| % énantiomère (-) | 0 | 3 | 6 | 9 |
|---|---|---|---|---|
| $T_{HOMO}$ (°C) | 31 | 32 | 33 | 34 |

<u>Conditions liées à la cinétique</u> :

- Température $T_I$ : 35°C
- Température $T_F$ : 18°C
- Température d'ensemencement

| Entraînement N° | Température d'ensemencement (°C) | Masse de l'ensemencement (mg) |
|---|---|---|
| 1 | 19,7 | 95 |
| 2 | 27,2 | 99 |
| 3 | 27,2 | 99 |
| 4 | 28,3 | 97 |

- Cinétique de refroidissement : T = f (T):

| t (min) | 0 | 15 | 30 | 45 |
|---|---|---|---|---|
| T (°C) | 35 | 29 | 24 | 18 |

Conditions initiale :

Excès énantiomérique initial : 9% ee.

| masse d'éthanol (g) | masse d'eau (g) | masse de (±) (g) | masse (-) (g) | masse de potasse (g) |
|---|---|---|---|---|
| 153,6 | 6,4 | 11,68 | 1,15 | 2,46 |

Résultats :

Filtration sur Büchner (diamètre 5 cm, 2 épaisseurs de papier filtre)

| Entraînement N° | masse d'énantiomère pur* (g) | masse équivalente de (S) Oméprazole (g) | excès énantiomérique (% ee) |
|---|---|---|---|
| 1 | 4,2 | 3,1 | (-) 93,2 |
| 2 | 6,8 | 4,9 | (+) 93,3 |
| 3 | 7,6 | 5,5 | (-) 91,7 |
| 4 | 7,5 | 5,4 | (+) 96,5 |
| **L'énantiomère pur correspond au solvate d'éthanol du sel de potassium du (S) Oméprazole* | | | |

Excès énantiomérique moyen : 93,6% ee.

**Exemple** 2 : **Dédoublement dans le mélange azéotropique éthanol/eau par cristallisation préférentielle autoensemencée**

Conditions liées aux équilibres :

Solubilité du mélange racémique dans le mélange azéotropique éthanol/eau contenant 1,2 équivalent molaire de potasse (soit un excès de potasse de 0,2 équivalent)

Coordonnées du point L : 8,7% massique ; température $T_L$ : 29°C

| Température (°C) | 29 | 31 | 35 |
|---|---|---|---|
| Solubilité (% massique) | 8,7 | 9,1 | 13,7 |

Évolution de $T_{HOMO}$ avec l'excès énantiomérique :

| % énantiomère (-) | 0 | 3 | 12 |
|---|---|---|---|
| $T_{HOMO}$ (°C) | 29 | 30 | 33 |

Conditions liées à la cinétique :

- Température $T_B$ : 30°C
- Température $T_F$ : 18°C
- Cinétique de refroidissement : T = f (T):

| t (min) | 0 | 15 | 30 | 45 |
|---|---|---|---|---|
| T (°C) | 30 | 25 | 18 | 18 |

Conditions initiales :

Excès énantiomérique initial : 9% ee.

| masse d'éthanol (g) | masse d'eau (g) | masse de (±) (g) | masse (-) (g) | masse de potasse (g) |
|---|---|---|---|---|
| 160 | 6,4 | 11,6 | 1,15 | 2,57 |

Résultats :

Filtration sur Büchner (diamètre 5 cm, 2 épaisseurs de papier filtre)

| Entraînement N° | masse d'énantiomère pur* (g) | Masse équivalente de (S) Oméprazole (g) | excès énantiomérique (% ee) |
|---|---|---|---|
| 1 | 2,1 | 1,5 | (-) 95,1 |
| 2 | 3,6 | 2,6 | (+) 93,4 |
| 3 | 4,1 | 3,0 | (-) 96,0 |
| 4 | 4,2 | 3,1 | (+) 90,3 |
| **L'énantiomère pur correspond au solvate d'éthanol du sel de potassium du (S) Oméprazole* | | | |

Excès énantiomérique moyen : 94% ee.

**Exemple 3 : Dédoublement dans le mélange azéotropique éthanol/eau par cristallisation préférentielle autoensemencée**

Conditions liées aux équilibres :

Solubilité du mélange racémique dans le mélange azéotropique éthanol/eau contenant 1,2 équivalent molaire de potasse (soit un excès de potasse de 0,2 équivalent)

Coordonnées du point L : 13,7% massique ; température $T_L$ : 35°C

| Température (°C) | 29 | 31 | 35 |
|---|---|---|---|
| Solubilité (% massique) | 8,7 | 9,1 | 13,7 |

Évolution de $T_{HOMO}$ avec l'excès énantiomérique :

| % énantiomère (-) | 0 | 3 | 6 | 9 |
|---|---|---|---|---|
| $T_{HOMO}$ (°C) | 35 | 36 | 37 | 38 |

Conditions liées à la cinétique :

- Température $T_B$ : 36°C
- Température $T_F$ : 25°C
- Cinétique de refroidissement : T = f (T):

| t (min) | 0 | 15 | 30 | 45 |
|---|---|---|---|---|
| T (°C) | 36 | 30 | 25 | 25 |

Conditions initiales :

Excès énantiomérique initial : 9% ee.

| masse d'éthanol (g) | masse d'eau (g) | masse de (±) (g) | masse de (-) (g) | masse de potasse (g) |
|---|---|---|---|---|
| 160 | 6,4 | 19 | 1,88 | 4,1 |

Résultats :

Filtration sur Büchner (diamètre 5 cm, 2 épaisseurs de papier filtre)

| Entraînement N° | masse d'énantiomère pur* (g) | masse équivalente de (S) Oméprazole (g) | excès énantiomérique (% ee) |
|---|---|---|---|
| 1 | 5,9 | 4,3 | (-) 84,1 |
| 2 | 7,0 | 5,1 | (+) 95,8 |
| 3 | 6,2 | 4,5 | (-) 94,0 |
| 4 | 6,1 | 4,4 | (+) 87,0 |
| **L'énantiomère pur correspond au solvate d'éthanol du sel de potassium du (S) Oméprazole* | | | |

Excès énantiomérique moyen : 90% ee.

**Exemple 4 : Dédoublement dans un mélange éthanol/eau par cristallisation préférentielle autoensemencée**

A partir de la liqueur mère issue de l'entraînement N°4 de l'exemple 3, une compensation est effectuée et de l'eau est ajoutée afin de d'augmenter la solubilité.

Conditions liées aux équilibres :

Le mélange éthanol/eau est estimé à 86/14 % masse/masse.
Evolution de $T_{HOMO}$ avec l'excès énantiomérique :

| % énantiomère (-) | 0 | 3 | 6 | 9 |
|---|---|---|---|---|
| $T_{HOMO}$ (°C) | 14 | 16 | 18 | 20 |

Conditions liées à la cinétique :

- Température $T_B$ : 15°C
- Température $T_F$ : 2°C
- Cinétique de refroidissement : T = f (T):

| t (min) | 0 | 15 | 30 | 45 |
|---|---|---|---|---|
| T (°C) | 15 | 10 | 5 | 2 |

Conditions initiales :

Excès énantiomérique initial : 8,2% ee.

| masse d'éthanol (g) | masse d'eau (g) | masse de (±) (g) | Masse de (-) (g) | masse de potasse (g) |
|---|---|---|---|---|
| 160 | 17,9 | 42,5 | 3,8 | 9,1 |

Résultats :

Filtration sur Büchner (diamètre 5 cm, 2 épaisseurs de papier filtre)

| Entraînement N° | masse d'énantiomère pur* (g) | masse équivalente de (S) Oméprazole (g) | Excès énantiomérique (% ee) |
|---|---|---|---|
| 1 | 6,4 | 4,8 | (-) 75,5 |
| 2 | 8,7 | 7,0 | (+) 80 |
| 3 | 13,2 | 12,3 | (-) 93,2 |
| 4 | 15,6 | 13,1 | (+) 83,9 |
| *L'énantiomère pur correspond au solvate d'éthanol du sel de potassium du (S) Oméprazole | | | |

Excès énantiomérique : 83% ee.

**Exemple 5 : Dédoublement dans un mélange éthanol/eau (90/10 % masse/masse) par cristallisation préférentielle autoensemencée à l'échelle de 2 litres**

Conditions liées aux équilibres :

Solubilité du mélange racémique dans le mélange éthanol/eau (90/10 % masse/masse) contenant 1,2 équivalent molaire de potasse (soit un excès de potasse de 0,2 équivalent):
Coordonnées du point L **:** 25 % massique ; température $T_L$ : 30°C

| Température (°C) | 30 |
|---|---|
| Solubilité (% massique) | 25 |

Evolution de $T_{HOMO}$ avec l'excès énantiomérique :

| % énantiomère (-) | 0 | 10 |
|---|---|---|
| $T_{HOMO}$ (°C) | 30 | 34,2 |

Conditions liées à la cinétique :

- Température $T_B$ : 31°C
- Température $T_F$ : 21°C
- Cinétique de refroidissement :

T = -1/3 t + 31 (entre 0 et 30 minutes, puis suivi d'un palier) :

| t (min) | 0 | 15 | 30 | 45 |
|---|---|---|---|---|
| T (°C) | 31 | 26 | 21 | 21 |

Conditions initiales :

Excès énantiomérique initial : 10% ee.

| masse d'éthanol (g) | masse d'eau (g) | masse de (±) (g) | masse de (-) (g) | masse de potasse (g) |
|---|---|---|---|---|
| 1440 | 160 | 388 + 5,1 (*) | 43,6 | 69,2 |
| (*) masse de mélange racémique additionnée suite à l'ajout d'énantiomère à 89,4% ee. | | | | |

Résultats :

La filtration a lieu sur une essoreuse (RA20 Rousselet-Robatel France) à 5000 rpm avec une chaussette de diamètre 200 mm, de hauteur 100 mm et dont le diamètre des pores du média filtrant en nylon est de 20 µm.

| Entraînement N° | masse d'énantiomère pur* (g) | masse équivalente de (S) Oméprazole (g) | Excès énantiomérique (% ee) | Compensation en éthanol |
|---|---|---|---|---|
| 1 | 125,2 | 90,9 | (+) 89,4 | - |
| 2 | 126,4 | 91,8 | (-) 97,2 | - |
| 3 | 157,7 | 114,5 | (+) 87,6 | - |
| 4 | 160,8 | 116,8 | (-) 94,6 | - |
| 5 | 222,7 | 161,7 | (+) 96,8 | - |
| **L'énantiomère pur correspond au solvate d'éthanol du sel de potassium du (S) Oméprazole. La masse reportée dans cette colonne est égale à la masse de la récolte multiplié par l'excès énantiomèrique.* | | | | |

Excès énantiomérique : 93,1% ee.

**Exemple 6 : Dédoublement dans un mélange éthanol/eau (93/7 % masse/masse) par cristallisation préférentielle autoensemencée à l'échelle de 2 litres**

Conditions liées aux équilibres :

Solubilité du mélange racémique dans le mélange **éthanol/eau** (93/7 % masse/masse) contenant 1,2 équivalent molaire de potasse (soit un excès de potasse de 0,2 équivalent):

Coordonnées du point L : 20% massique ; température $T_L$ : 34,4°C

Evolution de $T_{HOMO}$ avec l'excès énantiomérique :

| % énantiomère (-) | 0 | 8,4 |
|---|---|---|
| $T_{HOMO}$ (°C) | 34,4 | 37,1 |

Conditions liées à la cinétique :

- Température $T_B$ : 35°C
- Température $T_F$ : 25°C
- Cinétique de refroidissement :

T = -1/3 t + 35 (entre 0 et 30 minutes, puis suivi d'un palier) :

| t (min) | 0 | 15 | 30 | 45 |
|---|---|---|---|---|

(suite)

| T (°C) | 35 | 30 | 25 | 25 |
|---|---|---|---|---|

Conditions initiales :

Excès énantiomérique initial : 8,3% ee.

| masse d'éthanol (g) | masse d'eau (g) | masse de (±) (g) | masse de (-) (g) | masse de potasse (g) |
|---|---|---|---|---|
| 1440 | 110 | 271 | 24,8 | 55 |

Résultats :

La filtration a lieu sur une essoreuse (RA20 Rousselet-Robatel France) à 5000 rpm avec une chaussette de diamètre 200 mm, de hauteur 100 mm et dont le diamètre des pores du média filtrant en nylon est de 20 $\mu$m.

| Entraîne -ment N° | masse d'énantiomère pur* (g) | masse équivalente de (S) Oméprazole (g) | excès énantiomérique (% ee) | Compensation en éthanol (g) |
|---|---|---|---|---|
| 1 | 98,36 | 71,44 | (+) 89,4 | 45 |
| 2 | 119,59 | 86,86 | (-) 97,2 | - |
| 3 | 138,41 | 100,53 | (+) 87,6 | - |
| 4 | 132,40 | 96,16 | (-) 94,6 | 50 |
| 5 | 106,50 | 77,34 | (+) 96,8 | - |
| 6 | 133,30 | 96,82 | (-) 95,2 | - |
| 7 | 129,37 | 93,97 | (+) 93,8 | - |
| *L'énantiomère pur correspond au solvates d'éthanol du sel de potassium du (S) Oméprazole. La masse reportée dans cette colonne est égale à la masse de la récolte multipliée par l'excès énantiomèrique | | | | |

Excès énantiomérique moyen : 93,5% ee.

**Exemple 7 : Dédoublement dans l'éthanol par cristallisation préférentielle autoensemencée**

Conditions liées aux équilibres :

Solubilité du mélange racémique dans l'éthanol absolue contenant 1,2 équivalent molaire de potasse (soit un excès de potasse de 0,2 équivalent)

Coordonnées du point L **:** 2,2% massique ; température $T_L$ : 33°C

| Température (°C) | 26 | 30 | 33 |
|---|---|---|---|
| Solubilité (% massique) | 1,3 | 1,7 | 2,2 |

Évolution de $T_{HOMO}$ avec l'excès énantiomérique :

| % antipode (-) | 0 | 3 | 6 | 9 |
|---|---|---|---|---|
| $T_{HOMO}$ (°C) | 33,0 | 33,2 | 33,5 | 33,8 |

Conditions liées à la cinétique :

- Température $T_B$ : 33,2°C
- Température $T_F$ : 13°C
- Cinétique de refroidissement : T = f (T):

| t (min) | 0 | 30 | 45 | 60 |
|---|---|---|---|---|
| T (°C) | 33,2 | 15 | 13 | 13 |

Conditions initiales :

Excès énantiomérique initial : 9% ee.

| Masse d'éthanol (g) | masse de (±) (g) | Masse de (-) (g) | masse de potasse (g) |
|---|---|---|---|
| 160 | 2,58 | 0,256 | 0,51 |

Le dédoublement dans l'éthanol montre les plus mauvais résultats, par conséquent son étude n'a pas été poursuivie et aucune filtration n'a été effectuée. En effet, le temps nécessaire pour chaque opération d'entraînement est de l'ordre de 3 heures. La solubilité du sel est faible et la récolte ainsi obtenue est faible de l'ordre de 2,5g pour 1L de solution, ce qui nuit au rendement de chaque entraînement.

**Exemple 8 : Dédoublement dans le mélange éthanol/éthylèneglycol (80/20 % masse/masse) par cristallisation préférentielle autoensemencée**

Conditions liées aux équilibres :

Évolution de $T_{HOMO}$ avec l'excès énantiomérique :

| % énantiomère (-) | 0 | 3,2 | 6 | 9 |
|---|---|---|---|---|
| $T_{HOMO}$ (°C) | 33,3 | 34,3 | 35,1 | 35,6 |

Conditions liées à la cinétique :

- Température $T_B$ : 35°C
- Température $T_F$ : 18°C
- Cinétique de refroidissement : T = f (T):

| t (min) | 0 | 20 | 30 | 40 | 45 |
|---|---|---|---|---|---|
| T (°C) | 35 | 28 | 24 | 20 | 18 |

Conditions initiales :

Excès énantiomérique initial : 9% ee.

| masse d'éthanol (g) | masse d'éthylèneglycol (g) | masse de (±) (g) | masse (-) (g) | Masse de potasse (g) |
|---|---|---|---|---|
| 32 | 8 | 10,35 | 0,74 | 2,14 |

Résultats :

Filtration sur verre fritté N°3

| Entraînement N° | Masse d'énantiomère pur* (g) | excès énantiomérique (% ee) |
|---|---|---|
| 1 | 2,9 | (-) 70,0 |
| 2 | 2,3 | (+) 62,5 |
| 3 | 2,7 | (-) 63,8 |
| 4 | 3,5 | (+) 78,0 |

**[0134]** * L'énantiomère pur correspond à un mélange entre le solvate d'éthanol du sel de potassium du (S) Oméprazole et le solvate d'éthylèneglycol du sel de potassium du (S) Oméprazole

Excès énantiomérique moyen : 68,6% ee.

**Exemple 9** : **Structure du sel de potassium du *(S)* Oméprazole solvaté par l'éthanol**

Un monocristal a été obtenu dans une solution saturée en Oméprazole racémique préparée par dissolution de l'Oméprazole racémique dans une solution de potasse éthanolique. La nucléation puis la croissance du monocristal ont été induites par diminution de la température provoquant la sursaturation de la solution en sel.

La structure cristalline du monocristal a été résolue dans le système monoclinique, groupe d'espace $P2_1$. La maille cristalline élémentaire contient une molécule d'Oméprazole sous forme anionique, un cation potassium et deux molécules d'éthanol.

Les intensités de diffraction ont été mesurées avec un diffractomètre SMART APEX automatique (BRUKER) pourvu du logiciel SMART (SMART for WNT/2000 V5.622 (2001), Smart software référence manual, Bruker Advanced X Ray Solutions, Inc., Madison, Wisconsin, USA) et la structure a été résolue avec les logiciels SAINT+, SADABS et SHELXS (SAINT+ V6.02 (1999), Saint software référence manual, Bruker Advanced X Ray Solutions, Inc., Madison, Wisconsin, USA).

Le facteur de reliabilité R1 est de 3,09%, ce qui indique que la résolution est satisfaisante. La valeur du paramètre de Flack est de 0.11, ce qui permet de conclure que la molécule, dans le cristal étudié, est bien de configuration absolue (S).

Les caractéristiques cristallographiques de cette phase sont rassemblées dans le tableau V ci-dessous.

Tableau V

| Identification | Sel de potassium du (S) Oméprazole solvaté par 2 molécules d'éthanol |
|---|---|
| Formule Chimique | $C_{21} H_{30} K N_3 O_5 S_1$ |
| Masse Molaire (*g.mol$^{-1}$*) | 475.64 |
| Système Cristallin | Monoclinique |
| Groupe d'espace | $P2_1$ |
| Z, Z' | 2, 1 |
| a (*Å*) | 11.057 |
| b (*Å*) | 7,708 |
| c (*Å*) | 13.989 |
| α (°) | 90.0 |
| β (°) | 100.12 |
| γ (°) | 90.0 |
| V (*Å$^3$*) | 1173.8 |

La représentation 3D de la maille cristalline, construite à l'aide du logiciel DIAMOND, présentée sur la figure

6, fait apparaître l'anion benzimidazole, le cation potassium et 2 molécules d'éthanol. Il n'y a pas de lien électrostatique direct entre les atomes d'azote du noyau benzimidazole et le cation $K^+$. Le lien est effectué via le relais d'une molécule d'éthanol.

Les coordonnées des atomes dans la maille cristalline sont présentées dans le tableau VI ci-dessous.

Tableau VI : Coordonnées atomiques (x10^4) et paramètres de déplacement isotropique (A^2 x 10^3).

| | x | y | z | U(eq) |
|---|---|---|---|---|
| K(1) | 10723(1) | 7414(1) | 10034(1) | 40(1) |
| S(1) | 7268(1) | 6353(1) | 10330(1) | 32(1) |
| N(1) | 9493(2) | 9468(2) | 11377(1) | 37(1) |
| O(4) | 13316(1) | 7340(3) | 10314(1) | 48(1) |
| C(8) | 5798(2) | 7542(3) | 7719(1) | 36(1) |
| N(3) | 6824(1) | 7257(3) | 8417(1) | 38(1) |
| O(5) | 9024(2) | 9070(3) | 8606(1) | 55(1) |
| C(10) | 8449(2) | 8839(2) | 11601(1) | 32(1) |
| O(1) | 9268(2) | 8297(3) | 14226(1) | 62(1) |
| C(16) | 8317(2) | 8429(3) | 12556(1) | 37(1) |
| C(17) | 7142(2) | 7704(4) | 12789(2) | 53(1) |
| N(2) | 5106(1) | 6797(2) | 9104(1) | 38(1) |
| C(2) | 4742(2) | 7272(3) | 8141(1) | 36(1) |
| C(3) | 3573(2) | 7525(4) | 7593(2) | 49(1) |
| C(5) | 4528(2) | 8288(3) | 6221(2) | 48(1) |
| C(12) | 10435(2) | 9342(3) | 13063(2) | 45(1) |
| C(7) | 5695(2) | 8044(3) | 6747(2) | 47(1) |
| O(3) | 4283(2) | 8785(3) | 5261(1) | 71(1) |
| C(4) | 3484(2) | 8034(3) | 6640(2) | 51(1) |
| C(14) | 9334(2) | 8712(3) | 13279(1) | 42(1) |
| C(13) | 11563(2) | 9610(5) | 13825(2) | 69(1) |
| C(11) | 10450(2) | 9701(3) | 12099(2) | 40(1) |
| C(21) | 9206(2) | 9536(5) | 7663(2) | 69(1) |
| C(20) | 10438(3) | 9094(4) | 7475(2) | 61(1) |
| C(6) | 5298(3) | 8970(6) | 4771(2) | 81(1) |
| C(18) | 13537(3) | 7470(6) | 12043(2) | 79(1) |
| C(19) | 14043(2) | 6936(5) | 11210(2) | 75(1) |
| C(15) | 8819(4) | 9683(6) | 14746(2) | 90(1) |
| O(2) | 8510(1) | 5858(2) | 10129(1) | 39(1) |
| C(1) | 6326(2) | 6841(2) | 9189(1) | 32(1) |
| C(9) | 7425(2) | 8611(3) | 10747(1) | 36(1) |

Sur la figure 4 sont présentés les diffractogrammes de rayons X sur poudre expérimentaux des phases racémique et énantiomériquement pure et le diffractogramme de rayons X sur poudre calculé à partir de cette structure.

**__Exemple 10__ : __Préparation du sel de potassium du (*S*) Oméprazole à partir du sel de magnésium du (*S*) Oméprazole__**

Le sel de magnésium du (S) Oméprazole trihydrate (10,02g), disponible commercialement, est dissous dans 400mL d'éthanol auquel on ajoute de la potasse en excès (3,99g, 2,5 équivalents molaires). Le sel de potassium est cristallisé par évaporation partielle du solvant, puis filtré sur Büchner. Le produit obtenu (14,7g) est recristallisé dans 2 litres d'éthanol à 45°C. Une filtration à chaud est réalisée afin d'éliminer les impuretés insolubles en présence de potasse (0,87g, environ 0,5 équivalent molaire).

**__Exemple 11__ : __Purification énantiomérique__**

10,2g (sur les 10,88g récoltés) de sel de potassium du (S) Oméprazole (80% ee, soit une masse d'énantiomère pur (S) de 8,16g), obtenu par l'entraînement N°2 de l'exemple 4, sont mis en suspension dans 120g d'éthanol sous

agitation et thermostatés à 25°C, auxquels est ajouté un excès de potasse (0,11g, 0,1 équivalent molaire).

La température est diminuée progressivement jusqu'à ce que le pouvoir rotatoire de la liqueur mère soit proche de zéro. Le solide obtenu est filtré sur Büchner. Après séchage, la masse du sel de potassium récupéré est de 7,7g (pour 8,16g théoriquement récupérables), soit un rendement supérieur à 94%, avec une pureté énantiomérique supérieure à 99% ee (mesurée par chromatographie HPLC chirale).

**<u>Exemple 12</u>** : **<u>Purification énantiomérique</u>**

Les récoltes des opérations de dédoublement :

- Exemple 6 entraînement 2(-) : 119g (97% ee soit 115g d'énantiomère (-);
- Exemple 6 entraînement 4(-) : 135g (94% ee soit 126g d'énantiomère (-);
- Exemple 6 entraînement 6(-) : 133g (95% ee soit 126g d'énantiomère (-).

Soit au total 387g, c'est-à-dire 367g d'énantiomère (-), ont été mélangés et mis en suspension dans une solution de potasse éthanolique de 2 litres, de telle sorte que la quantité de potasse correspond à 0,1 équivalent molaire par rapport à l'Oméprazole (≈4,5g ; l'éthanol de départ était de l'éthanol absolu).

La température est portée à 50°C pendant une demie heure puis est ramenée à 30°C rapidement et jusqu'à 14°C lentement en suivant par polarimétrie l'évolution du pouvoir rotatoire de la liqueur mère.

Lorsque le domaine triphasé est atteint (i.e. le pouvoir rotatoire est nul), la température est remontée à 16°C pendant 12 heures afin d'atteindre l'équilibre thermodynamique dans le domaine diphasé (énantiomère (-) du diéthanolate du sel de potassium de l'Oméprazole et sa solution saturée). Une fois à l'équilibre, la température est abaissée à 13°C pour avoir un effet d'entraînement et récupérer une masse d'énantiomère supérieure.

Auprès ½ heure, La filtration a lieu sur une essoreuse (RA20 Rousselet-Robatel France) à 5000 tours/min avec une chaussette de diamètre 200 mm, de hauteur 100 mm et dont le diamètre des pores du média filtrant en nylon est de 20 μm.

La masse récoltée est de 352g.

Une analyse HPLC a été réalisée afin de mesurer la pureté optique de l'échantillon après recristallisation.

La pureté est de 99,4% ee et le rendement est de 95%.

**<u>Exemple 13 : Préparation du solvate d'éthylèneglycol du sel de potassium de l'Oméprazole racémique</u>**

L'Oméprazole racémique (1,01g) est dissous dans un mélange de méthanol et d'éthylèneglycol (respectivement 4 mL et 1mL) en présence de potasse (180mg, 1,1 équivalents molaires). Après 3 heures d'agitation à température ambiante le solide est récupéré par filtration sur Büchner. Le solide obtenu correspond à un mélange de phases entre le solvate de méthanol et le solvate d'éthylèneglycol du sel de potassium de l'Oméprazole racémique. Après séchage, le solvate de méthanol devient amorphe, la seule phase observée par diffraction des RX sur poudre est le solvate d'éthylèneglycol. Le diffractogramme (XRPD) est présenté sur la figure 5.

**<u>Exemple 14 : Préparation du solvate d'éthylèneglycol du sel de potassium du (*S*) Oméprazole,</u>**

Le solvate d'éthanol du sel de potassium du (S) Oméprazole (3,01g), obtenu par recristallisation de la récolte de l'entraînement 1 de l'exemple 4 par le même protocole qu'à l'exemple 12, est mis en suspension dans un mélange de solvants de méthanol et d'éthylèneglycol (respectivement 2mL et 1mL). Après 5 jours d'agitation à température ambiante, la suspension est récupérée par filtration sur fritté N°3. Le solide obtenu est un mélange de phases entre le solvate de méthanol et le solvate d'éthylèneglycol du sel de potassium du (S) Oméprazole. Après séchage à atmosphère ambiante, le solvate de méthanol devient amorphe, la seule phase observée par diffraction des RX sur poudre est le solvate d'éthylèneglycol. Le diffractogramme (XRPD) est présenté sur la figure 5.

## Revendications

1. Procédé de dédoublement de sels de l'Oméprazole racémique, **caractérisé en ce que** l'on transforme l'Oméprazole racémique en sel de potassium sous forme de solvates d'éthanol ou d'éthylène glycol ou d'un mélange de ceux-ci, en présence d'un excès de base minérale source de potassium, lesdits sels de potassium de l'Oméprazole racémique sous forme de solvates se présentant sous la forme de conglomérats dont les domaines de solution solide partielle s'ils existent, sont inférieurs à 1%, puis **en ce que** l'on dédouble lesdits conglomérats par cristallisation préférentielle pour séparer les deux énantiomères (S) et (R) dudit sel de potassium de l'Oméprazole racémique.

**2.** Procédé selon la revendication 1, **caractérisé en ce que** le dédoublement des conglomérats est réalisé par cristallisation préférentielle ensemencée ou autoensemencée.

**3.** Procédé selon l'une quelconque des revendications précédentes, **caractérisé en ce que** la cristallisation préférentielle est mise en oeuvre avec un système choisi parmi :

- des systèmes quinaires [$K_2O$ - EtOH - $H_2O$ - (-) Oméprazole - (+) Oméprazole] et [$K_2O$ - Ethylèneglycol - $H_2O$ - (-) Oméprazole - (+) Oméprazole] ;
- un système sénaire [$K_2O$ - EtOH - Ethylèneglycol - $H_2O$ -(-) Oméprazole - (+) Oméprazole] ;
- des systèmes ternaires, par assimilation des systèmes quinaires à des systèmes ternaires comprenant les constituants **:** alcool(s) (éthanol et/ou éthylèneglycol), eau et excès de potasse dans le médium.

**4.** Procédé selon l'une quelconque des revendications 1 à 3, **caractérisé en ce que** la cristallisation préférentielle est ensemencée et **en ce qu'**il comprend les étapes suivantes :

a) on prépare une première solution homogène composée du mélange racémique sous forme de conglomérat et d'un excès du premier énantiomère à récupérer sous forme de solvate du sel de potassium du (X) Oméprazole, désigné X-K-solvate, où X représente l'énantiomère (R) ou (S), et d'un médium, dont le point figuratif I, défini par les variables concentration et température $T_I$ ($T_I > T_{HOMO}$), se situe dans le domaine monophasé composé de la solution sous saturée ;
b) on applique une loi de programmation de refroidissement au mélange monophasé ;
c) lorsque le mélange atteint une température inférieure à la température $T_{HOMO}$, on ensemence la solution avec des germes énantiomériquement purs du premier énantiomère X-K-solvate à récupérer.
d) on adapte pendant toute la durée de la croissance cristalline une vitesse d'agitation légèrement croissante en fonction du temps de façon à ce que celle-ci soit suffisamment lente pour favoriser une croissance du premier énantiomère X-K-solvate ;
e) on récolte les cristaux du premier énantiomère X-K-solvate ;
f) on ajoute aux liqueurs mères la même masse de mélange racémique que la masse de la récolte réalisée à l'étape précédente, et on porte le nouvel ensemble à la température $T_I$ ($T_I > T_{HOMO}$), le point I' se situant dans le domaine monophasé ;
g) on applique la même loi de programmation de refroidissement qu'à l'étape (b) au mélange monophasé préparé à l'étape (f) contenant le second énantiomère, de sorte que les liqueurs mères gardent une faible sursaturation pendant la cristallisation afin de privilégier la croissance du second énantiomère X-K-solvate lors de l'ensemencement;
h) lorsque le mélange atteint une température inférieure à la température $T_{HOMO}$, on ensemence la solution avec des germes énantiomériquement purs du second énantiomère X-K-solvaté ;
i) on adapte pendant toute la durée de la croissance cristalline de l'étape précédente, une vitesse d'agitation légèrement croissante en fonction du temps de façon à ce que celle-ci soit suffisamment lente pour favoriser la croissance de ce second énantiomère X-K-solvate;
j) on récolte les cristaux du second énantiomère X-K-solvate ;
k) on ajoute aux liqueurs mères la même masse de mélange racémique que la masse de la récolte réalisée à l'étape précédente, pour obtenir une solution dont la composition est identique à celle de la solution initiale ;
l) on répète les étapes (a) à (k) pour obtenir successivement l'un puis l'autre des deux énantiomères.

**5.** Procédé selon l'une quelconque des revendications 1 à 3, **caractérisé en ce que** la cristallisation préférentielle est auto-ensemencée et **en ce qu'**il comprend les étapes suivantes :

a) on prépare un premier ensemble composé du mélange racémique sous forme de conglomérat, du premier énantiomère à récupérer, sous forme de solvate du sel de potassium du (X) Oméprazole, désigné X-K-solvate, où X représente l'énantiomère (R) ou (S), et d'un médium, dont le point figuratif E, défini par les variables concentration et température $T_B$, se situe dans le domaine biphasé composé de cristaux de X-K-solvate et de sa solution saturée ;
b) on applique une loi de programmation de refroidissement au mélange biphasé, telle que les liqueurs mères gardent une faible sursaturation qui privilégie la croissance du premier énantiomère X-K-solvate présent sous forme de cristaux, tout en interdisant la nucléation spontanée du second énantiomère X-K-solvate dissout dans la solution ;
c) on adapte pendant toute la durée de la croissance cristalline une vitesse d'agitation légèrement croissante en fonction du temps de façon à ce que celle-ci soit suffisamment lente pour favoriser une croissance du premier

énantiomère X-K-solvate, en évitant de générer une nucléation non maîtrisée et l'attrition de cristaux ;
d) on récolte les cristaux du premier énantiomère X-K-solvate ;
e) on ajoute aux liqueurs mères la même masse de mélange racémique que la masse de la récolte réalisée à l'étape précédente, et on porte le nouvel ensemble à la température $T_B$, le point E' se situant dans le domaine biphasé du second énantiomère X-K-solvate en excès, en équilibre avec sa solution saturée;
f) on applique la même loi de programmation de refroidissement qu'à l'étape (b) au mélange biphasé préparé à l'étape (e) contenant le second énantiomère X-K-solvate, de sorte que les liqueurs mères gardent une faible sursaturation pendant la cristallisation afin de privilégier la croissance du second énantiomère X-K-solvate présent sous forme de cristaux tout en interdisant la nucléation spontanée du premier énantiomère X-K-solvate présent dans la solution ;
g) on adapte pendant toute la durée de la croissance cristalline de l'étape précédente, une vitesse d'agitation légèrement croissante en fonction du temps de façon à ce que celle-ci soit suffisamment lente pour favoriser la croissance de ce second énantiomère X-K-solvate en évitant de générer une nucléation non maîtrisée et l'attrition de cristaux ;
h) on récolte les cristaux du second énantiomère X-K-solvate ;
i) on ajoute aux liqueurs mères la même masse de mélange racémique que la masse de la récolte réalisée à l'étape précédente, pour obtenir un ensemble dont la composition est identique à celle de l'ensemble E initial ;
j) on répète les étapes (a) à (i) pour obtenir successivement l'un puis l'autre des deux énantiomères.

6. Procédé selon l'une quelconque des revendications 4 ou 5, **caractérisé en ce qu'**à l'étape (a), le médium est constitué par un solvant alcoolique ou mélange de solvants alcooliques, de l'eau et un excès de potasse.

7. Procédé selon la revendication 6, **caractérisé en ce que** le solvant alcoolique ou le mélange de solvants alcooliques est choisi parmi l'éthanol, l'éthylèneglycol, pur ou en présence d'eau.

8. Procédé selon l'une quelconque des revendications précédentes, **caractérisé en ce qu'**il comprend une étape de recyclage de l'énantiomère (R) suivant les étapes suivantes :

i) Réduction de ce sulfoxyde chiral (R) en sulfure (thio-éther) achiral.
ii) Oxydation de l'atome de soufre de ce sulfure en sulfoxyde racémique, à l'aide d'eau oxygénée ou d'ion hypochlorite ou d'acide perbenzoïque ou tout autre oxydant en évitant la formation de sulfone.

**Patentansprüche**

1. Verfahren zur Trennung von Salzen von racemischem Omeprazol, **dadurch gekennzeichnet, dass** das racemische Omeprazol in Gegenwart eines Überschusses an einer anorganischen Base, die eine Kaliumquelle ist, in ein Kaliumsalz in Form von Ethanol- oder Ethylenglycolsolvaten oder eines Gemisches davon umgewandelt wird, wobei die Kaliumsalze des racemischen Omeprazols in Form von Solvaten in Form von Konglomeraten vorliegen, deren Bereiche an teilweise fester Lösung, falls vorhanden, weniger als 1% betragen, dass anschließend die Konglomerate durch selektive Kristallisation getrennt werden, um die beiden Enantiomere (S) und (R) des Kaliumsalzes des racemischen Omeprazols zu trennen.

2. Verfahren nach Anspruch 1, **dadurch gekennzeichnet, dass** die Trennung der Konglomerate durch geimpfte oder autogeimpfte selektive Kristallisation durchgeführt wird.

3. Verfahren nach einem der vorhergehenden Ansprüche, **dadurch gekennzeichnet, dass** die selektive Kristallisation mit einem System durchgeführt wird, das ausgewählt ist aus:

- quinären Systemen [$K_2O$ - EtOH - $H_2O$ - (-) Omeprazol - (+) Omeprazol] und [$K_2O$ - Ethylenglycol - $H_2O$ - (-) Omeprazol - (+) Omeprazol];
- einem senären System [$K_2O$ - EtOH - Ethylenglycol - $H_2O$ - (-) Omeprazol - (+) Omeprazol];
- ternären Systemen, durch Assimilierung von quinären Systemen mit ternären Systemen, umfassend die Bestandteile: Alkohol(e) (Ethanol und/oder Ethylenglycol), Wasser und einen Überschuss an Kaliumhydroxid in dem Medium.

4. Verfahren nach einem der Ansprüche 1 bis 3, **dadurch gekennzeichnet, dass** die selektive Kristallisation geimpft ist und dass sie die folgenden Schritte umfasst:

a) Herstellen einer ersten homogenen Lösung, zusammengesetzt aus dem racemischen Gemisch in Form eines Konglomerats und einem Überschuss des ersten zu gewinnenden Enantiomers in Form eines Solvats des Kaliumsalzes von (X) Omeprazol, bezeichnet als X-K-Solvat, wobei X das Enantiomer (R) oder (S) bedeutet, und einem Medium, dessen figurativer Punkt I, definiert durch die Variablen Konzentration und Temperatur $T_I$ ($T_I > T_{HOMO}$), sich im einphasigen Bereich befindet, zusammengesetzt aus der untersättigten Lösung;
b) Anwenden eines Kühlprogramms auf das einphasige Gemisch;
c) wenn das Gemisch eine Temperatur erreicht, die niedriger ist als die Temperatur $T_{HOMO}$, Beimpfen der Lösung mit enantiomerenreinen Keimen des zu gewinnenden ersten Enantiomers X-K-Solvat;
d) während der gesamten Dauer des kristallinen Wachstums, Einstellen einer in Abhängigkeit von der Zeit leicht ansteigenden Rührgeschwindigkeit, so dass diese ausreichend langsam ist, um ein Wachstum des ersten Enantiomers X-K-Solvat zu begünstigen;
e) Gewinnen der Kristalle des ersten Enantiomers X-K-Solvat;
f) Zugabe zu den Mutterlaugen der gleichen Menge des racemischen Gemischs wie die Menge der im vorhergehenden Schritt durchgeführten Gewinnung und Erwärmen der neuen Zusammensetzung auf die Temperatur $T_I$ ($T_I > T_{HOMO}$), wobei sich der Punkt I' im einphasigen Bereich befindet;
g) Anwenden des gleichen Kühlprogramms wie in Schritt (b) auf das in Schritt (f) hergestellte einphasige Gemisch, das das zweite Enantiomer enthält, so dass die Mutterlaugen während der Kristallisation eine schwache Übersättigung beibehalten, so dass das Wachstum des zweiten Enantiomers X-K-Solvat während der Beimpfung begünstigt wird;
h) wenn das Gemisch eine Temperatur erreicht, die niedriger ist als die Temperatur $T_{HOMO}$, Beimpfen der Lösung mit enantiomerenreinen Keimen des zweiten Enantiomers X-K-Solvat;
i) während der gesamten Dauer des kristallinen Wachstums des vorhergehenden Schritts, Einstellen einer in Abhängigkeit von der Zeit leicht ansteigenden Rührgeschwindigkeit, so dass diese ausreichend langsam ist, um das Wachstum des zweiten Enantiomers X-K-Solvat zu begünstigen;
j) Gewinnen der Kristalle des zweiten Enantiomers X-K-Solvat;
k) Zugabe zu den Mutterlaugen der gleichen Menge des racemischen Gemischs wie die Menge der im vorhergehenden Schritt durchgeführten Gewinnung, um eine Lösung zu erhalten, deren Zusammensetzung mit der der ursprünglichen Lösung identisch ist;
l) Wiederholen der Schritte (a) bis (k), um nacheinander das eine und anschließend das andere der beiden Enantiomere zu erhalten.

5. Verfahren nach einem der Ansprüche 1 bis 3, **dadurch gekennzeichnet, dass** die selektive Kristallisation autogeimpft ist und dass sie die folgenden Schritte umfasst:

a) Herstellen einer ersten Zusammensetzung, zusammengesetzt aus dem racemischen Gemisch in Form eines Konglomerats, dem ersten zu gewinnenden Enantiomer in Form eines Solvats des Kaliumsalzes von (X) Omeprazol, bezeichnet als X-K-Solvat, wobei X das Enantiomer (R) oder (S) bedeutet, und einem Medium, dessen figurativer Punkt E, definiert durch die Variablen Konzentration und Temperatur $T_B$, sich im zweiphasigen Bereich befindet, zusammengesetzt aus Kristallen des X-K-Solvats und seiner gesättigten Lösung;
b) Anwenden eines Kühlprogramms auf das zweiphasige Gemisch, so dass die Mutterlaugen eine schwache Übersättigung beibehalten, die das Wachstum des ersten Enantiomers X-K-Solvat, das in Form von Kristallen vorliegt, begünstigt, während die spontane Keimbildung des in der Lösung gelösten zweiten Enantiomers X-K-Solvat verhindert wird;
c) während der gesamten Dauer des kristallinen Wachstums, Einstellen einer in Abhängigkeit von der Zeit leicht ansteigenden Rührgeschwindigkeit, so dass diese ausreichend langsam ist, um ein Wachstum des ersten Enantiomers X-K-Solvat zu begünstigen, während das Auftreten einer unkontrollierten Keimbildung und der Abrieb von Kristallen vermieden werden;
d) Gewinnen der Kristalle des ersten Enantiomers X-K-Solvat;
e) Zugabe zu den Mutterlaugen der gleichen Menge des racemischen Gemischs wie die Menge der im vorhergehenden Schritt durchgeführten Gewinnung und Erwärmen der neuen Zusammensetzung auf die Temperatur $T_B$, wobei sich der Punkt E' im zweiphasigen Bereich des im Überschuss, im Gleichgewicht mit seiner gesättigten Lösung, vorliegenden zweiten Enantiomers X-K-Solvat befindet;
f) Anwenden des gleichen Kühlprogramms wie in Schritt (b) auf das in Schritt (e) hergestellte zweiphasige Gemisch, das das zweite Enantiomer X-K-Solvat enthält, so dass die Mutterlaugen während der Kristallisation eine schwache Übersättigung beibehalten, um das Wachstum des zweiten Enantiomers X-K-Solvat, das in Form von Kristallen vorliegt, zu begünstigen, während die spontane Keimbildung des in der Lösung vorhandenen ersten Enantiomers X-K-Solvat verhindert wird;
g) während der gesamten Dauer des kristallinen Wachstums des vorhergehenden Schritts Einstellen einer in

Abhängigkeit von der Zeit leicht ansteigenden Rührgeschwindigkeit, so dass diese ausreichend langsam ist, um das Wachstum des zweiten Enantiomers X-K-Solvat zu begünstigen, während das Auftreten einer unkontrollierten Keimbildung und der Abrieb von Kristallen vermieden werden;
h) Gewinnen der Kristalle des zweiten Enantiomers X-K-Solvat;
i) Zugabe zu den Mutterlaugen der gleichen Menge des racemischen Gemischs wie die Menge der im vorhergehenden Schritt durchgeführten Gewinnung, um eine Zusammensetzung zu erhalten, deren Zusammensetzung mit der der ursprünglichen Zusammensetzung E identisch ist;
j) Wiederholen der Schritte (a) bis (i), um nacheinander das eine und anschließend das andere der beiden Enantiomere zu erhalten.

**6.** Verfahren nach einem der Ansprüche 4 oder 5, **dadurch gekennzeichnet, dass** im Schritt (a) das Medium aus einem alkoholischen Lösungsmittel oder einem Gemisch alkoholischer Lösungsmittel, Wasser und einem Überschuss an Kaliumhydroxid besteht.

**7.** Verfahren nach Anspruch 6, **dadurch gekennzeichnet, dass** das alkoholische Lösungsmittel oder das Gemisch alkoholischer Lösungsmittel aus Ethanol, Ethylenglycol, rein oder in Gegenwart von Wasser, ausgewählt ist.

**8.** Verfahren nach einem der vorhergehenden Ansprüche, **dadurch gekennzeichnet, dass** es einen Schritt des Rückführens des Enantiomers (R) im Anschluss an die folgenden Schritte umfasst:

i) Reduktion des chiralen Sulfoxids (R) zu achiralem (Thioether)sulfid;
ii) Oxidation des Schwefelatoms des Sulfids zu racemischem Sulfoxid mit Hilfe von Wasserstoffperoxid oder Hypochlorition oder Perbenzoesäure oder einem anderen Oxidationsmittel unter Vermeidung der Bildung von Sulfon.

## Claims

**1.** Method for the resolution of salts of racemic omeprazole, **characterized in that** racemic omeprazole is converted to potassium salt in the form of ethanol solvate or ethylene glycol solvate or a mixture thereof, in the presence of an excess of inorganic base which is a source of potassium, the said potassium salts of racemic omeprazole in the form of solvates existing in the form of conglomerates, the partial solid solution regions of which, if they exist, are less than 1%, and then **in that** the said conglomerates are resolved by preferential crystallization in order to separate the two (S) and (R) enantiomers of the said potassium salt of racemic omeprazole.

**2.** Method according to claim 1, **characterized in that** the conglomerates are resolved by seeded or auto-seeded preferential crystallization.

**3.** Method according to any one of the preceding claims, **characterized in that** the preferential crystallization is carried out with a system chosen from:

- quinary systems [$K_2O$-EtOH-$H_2O$-(-)-omeprazole-(+)-omeprazole] and [K,O-ethylene glycol-H,O-(-)-omeprazole-(+)-omeprazole];
- a senary system [$K_2O$-EtOH-ethylene glycol-$H_2O$-(-)-omeprazole-(+)-omeprazole];
- ternary systems, by classification of the quinary systems as ternary systems comprising the constituents: alcohol(s) (ethanol and/or ethylene glycol), water and excess of potassium hydroxide in the medium.

**4.** Method according to any one of Claims 1 to 3, **characterized in that** the preferential crystallization is seeded and **in that** it comprises the following stages:

a) a first homogeneous solution is prepared which is composed of the racemic mixture in the conglomerate form and of an excess of the first enantiomer to be recovered in the form of a solvate of the potassium salt of (X)-omeprazole, denoted X-K-solvate, where X represents the (R) or (S) enantiomer, and of a medium, the representational point I of which, defined by the variables of concentration and temperature $T_I$ ($T_I > T_{HOMO}$), lies within the single-phase region composed of the undersaturated solution;
b) a cooling programming law is applied to the single-phase mixture;
c) when the mixture reaches a temperature below the temperature $T_{HOMO}$, the solution is seeded with enantiomerically pure seeds of the first X-K-solvate enantiomer to be recovered;

d) throughout the duration of the crystal growth, a stirring rate which gently increases as a function of the time is adjusted so that it is sufficiently slow to promote growth of the first X-K-solvate enantiomer;
e) the crystals of the first X-K-solvate enantiomer are harvested;
f) the same weight of racemic mixture as the weight of the crystals harvested in the preceding stage is added to the mother liquors and the new combination is brought to the temperature $T_I$ ($T_I > T_{HOMO}$), the point I' lying in the single-phase region;
g) the same cooling programming law as in stage (b) is applied to the single-phase mixture prepared in stage (f) comprising the second enantiomer, so that the mother liquors retain a slight supersaturation during the crystallization in order to favour the growth of the second X-K-solvate enantiomer during the seeding;
h) when the mixture reaches a temperature below the temperature $T_{HOMO}$, the solution is seeded with enantiomerically pure seeds of the second X-K-solvate enantiomer;
i) throughout the duration of the crystal growth of the preceding stage, a stirring rate which gently increases as a function of the time is adjusted so that it is sufficiently slow to promote the growth of this second X-K-solvate enantiomer;
j) the crystals of the second X-K-solvate enantiomer are harvested;
k) the same weight of racemic mixture as the weight of the crystals harvested in the preceding stage is added to the mother liquors in order to obtain a solution having an identical composition to that of the initial solution;
l) stages (a) to (k) are repeated in order to successively obtain one and then the other of the two enantiomers.

**5.** Method according to any one of Claims 1 to 3, **characterized in that** the preferential crystallization is auto-seeded and **in that** it comprises the following stages:

a) a first combination is prepared which is composed of the racemic mixture in the conglomerate form, of the first enantiomer to be recovered, in the form of a solvate of the potassium salt of (X)-omeprazole, denoted X-K-solvate, where X represents the (R) or (S) enantiomer, and of a medium, the representational point E of which, defined by the variables of concentration and temperature $T_B$, lies within the two-phase region composed of crystals of X-K-solvate and of its saturated solution;
b) a cooling programming law is applied to the two-phase mixture, such that the mother liquors retain a slight supersaturation which favours the growth of the first X-K-solvate enantiomer present in the form of crystals, while preventing the spontaneous nucleation of the second X-K-solvate enantiomer dissolved in the solution;
c) throughout the duration of the crystal growth, a stirring rate which gently increases as a function of the time is adjusted so that it is sufficiently slow to promote growth of the first X-K-solvate enantiomer, while avoiding the generation of uncontrolled nucleation and the attrition of crystals;
d) the crystals of the first X-K-solvate enantiomer are harvested;
e) the same weight of racemic mixture as the weight of the crystals harvested in the preceding stage is added to the mother liquors and the new combination is brought to the temperature $T_B$, the point E' lying in the two-phase region of the second X-K-solvate enantiomer in excess, in equilibrium with its saturated solution;
f) the same cooling programming law as in stage (b) is applied to the two-phase mixture prepared in stage (e) comprising the second X-K-solvate enantiomer, so that the mother liquors retain a slight supersaturation during the crystallization, in order to favour the growth of the second X-K-solvate enantiomer present in the form of crystals, while preventing the spontaneous nucleation of the first X-K-solvate enantiomer present in the solution;
g) throughout the duration of the crystal growth of the preceding stage, a stirring rate which gently increases as a function of the time is adjusted so that it is sufficiently slow to promote the growth of this second X-K-solvate enantiomer while avoiding the generation of uncontrolled nucleation and the attrition of crystals;
h) the crystals of the second X-K-solvate enantiomer are harvested;
i) the same weight of racemic mixture as the weight of the crystals harvested in the preceding stage is added to the mother liquors in order to obtain a combination having an identical composition to that of the initial combination E;
j) stages (a) to (i) are repeated in order to successively obtain one and then the other of the two enantiomers.

**6.** Method according to either one of Claims 4 and 5, **characterized in that**, in stage (a), the medium is composed of an alcoholic solvent or mixture of alcoholic solvents, water and an excess of potassium hydroxide.

**7.** Method according to Claim 6, **characterized in that** the alcoholic solvent or the mixture of alcoholic solvents is chosen from ethanol or ethylene glycol, pure or in the presence of water.

**8.** Method according to any one of the preceding claims, **characterized in that** it comprises a stage of recycling the (R) enantiomer according to the following stages:

i) Reduction of this chiral (R) sulphoxide to give the achiral sulphide (thioether);
ii) Oxidation of the sulphur atom of this sulphide to give the racemic sulphoxide, using aqueous hydrogen peroxide solution or hypochlorite ion or perbenzoic acid or any other oxidizing agent, while avoiding the formation of sulphone.

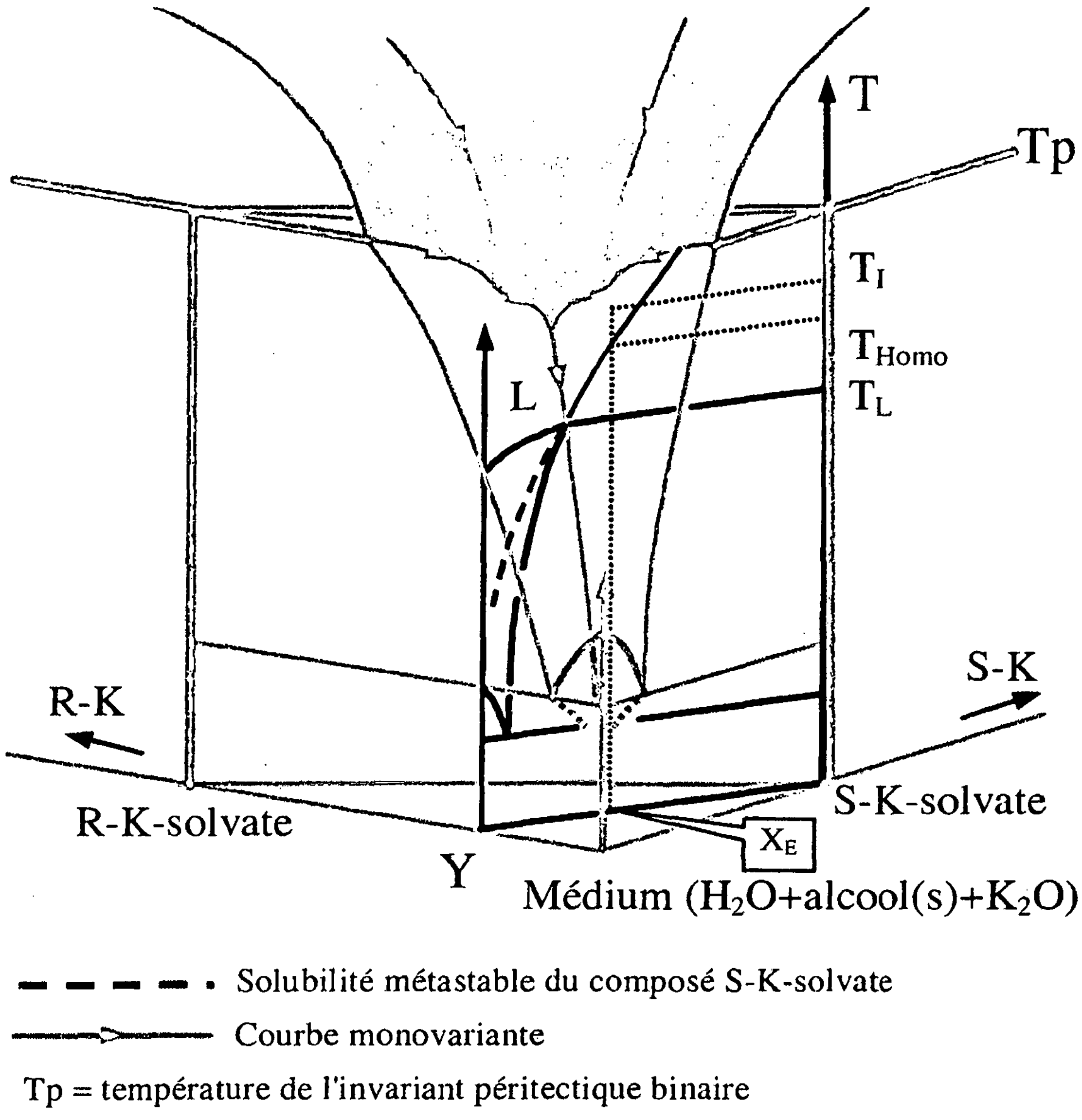


Figure 1 : Représentation en perspective d'une partie du système ternaire Médium - Enantiomère (S) - Enantiomère (R)

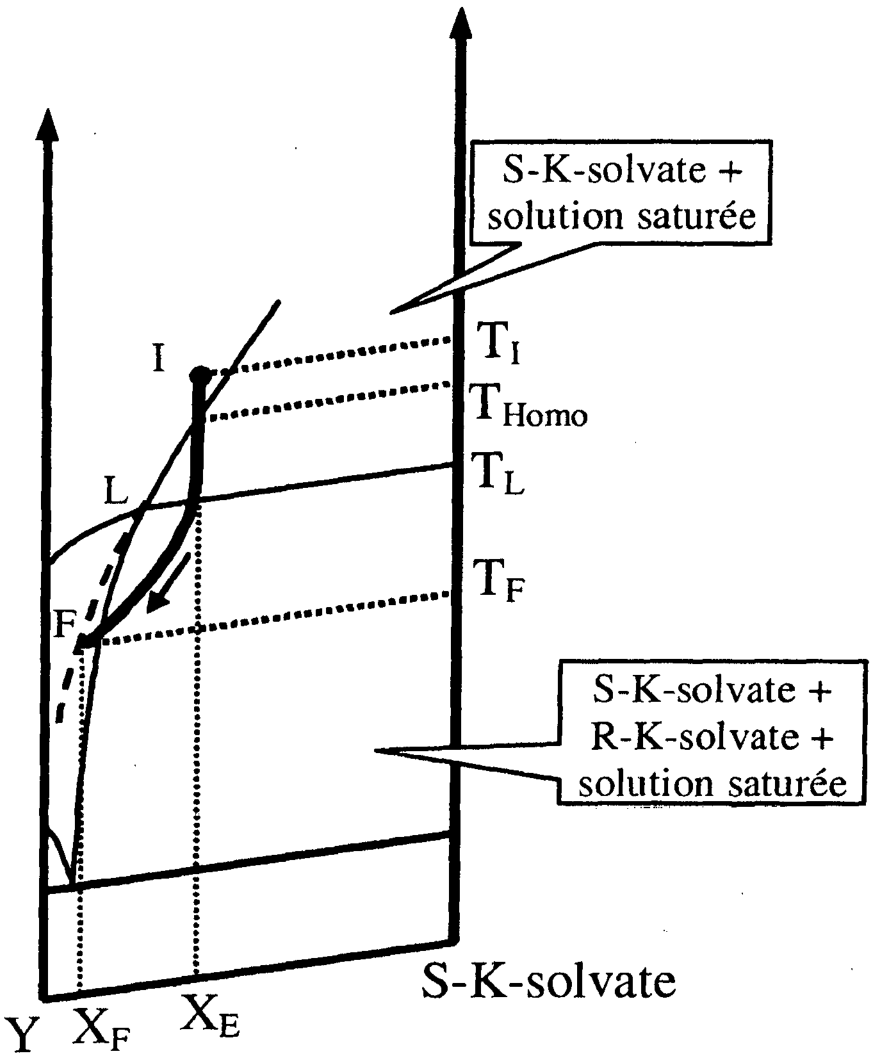


Figure 2 : Coupe verticale isoplèthe polythermique
Évolution de la composition de la liqueur mère au cours d'un entraînement dans le cas du procédé ensemencé.

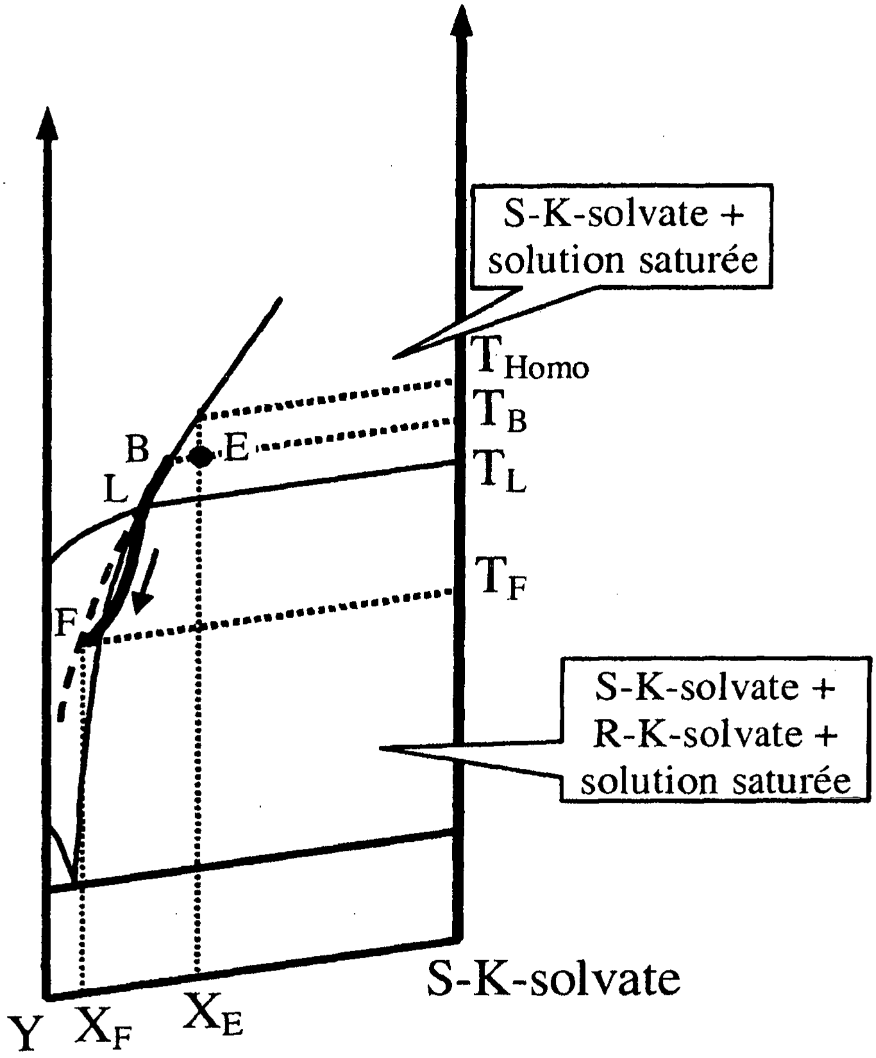


Figure 2bis : Coupe verticale isoplèthe polythermique Évolution de la composition de la liqueur mère au cours d'un entraînement dans le cas du procédé autoensemencé.

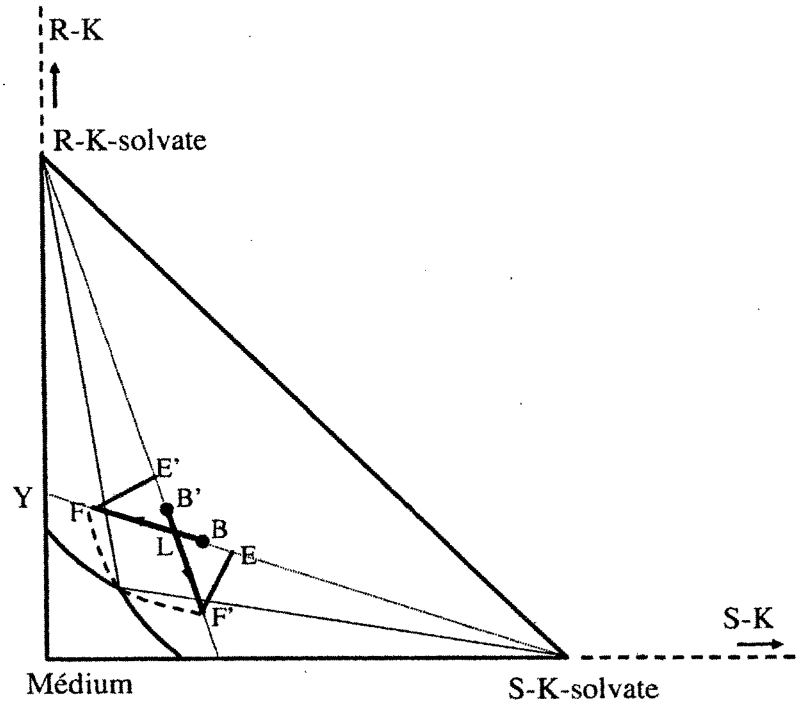


Figure 3 : Projection d'une partie du système ternaire
Médium - Enantiomère (S) - Enantiomère (R)
de la figure 1 sur le plan des concentrations
Évolution de la composition de la liqueur mère au cours d'un
entraînement dans le cas du procédé autoensemencé.

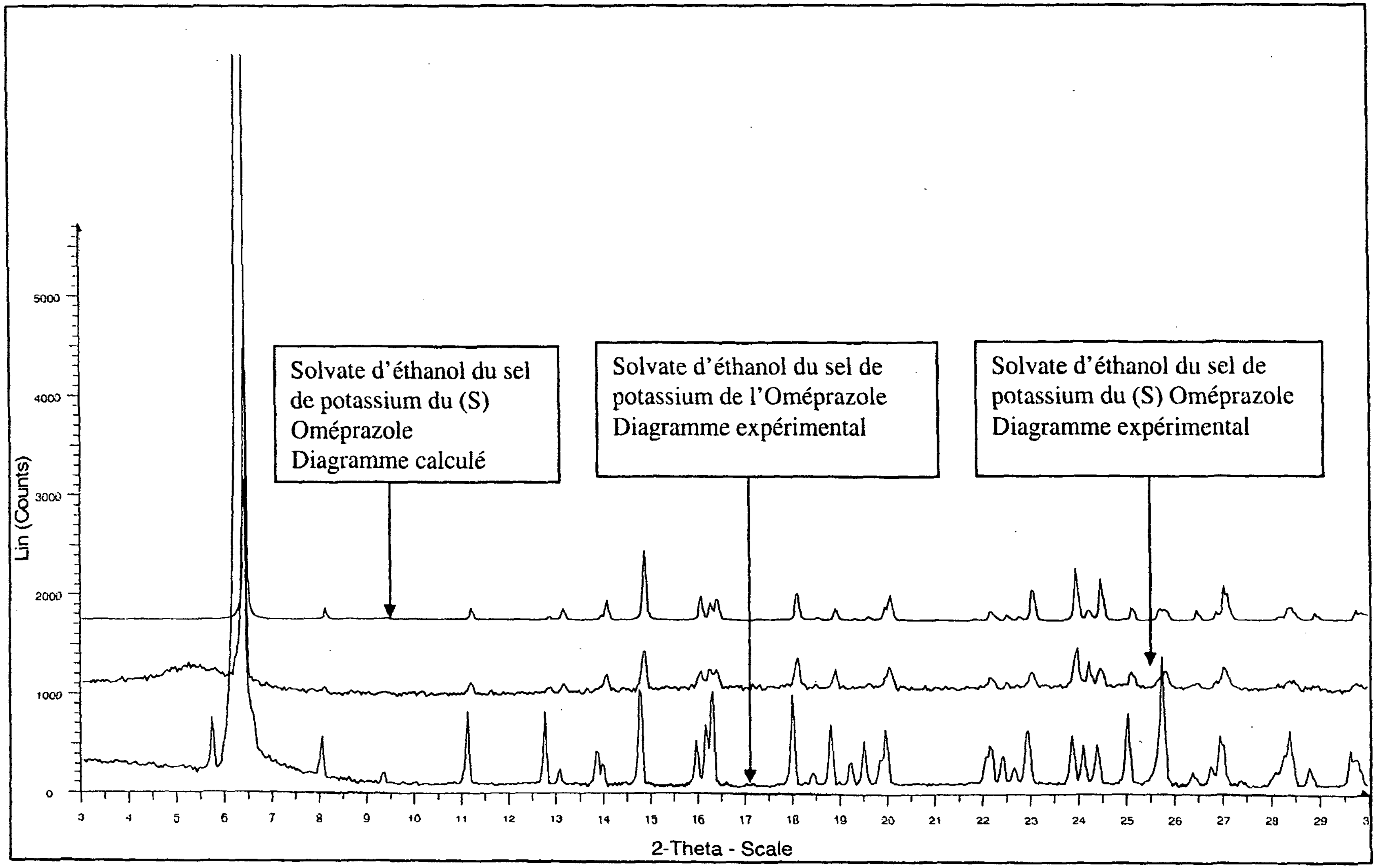


Figure 4 : Diffractogrammes du solvate d'éthanol du sel de potassium de l'Oméprazole et du (S) Oméprazole

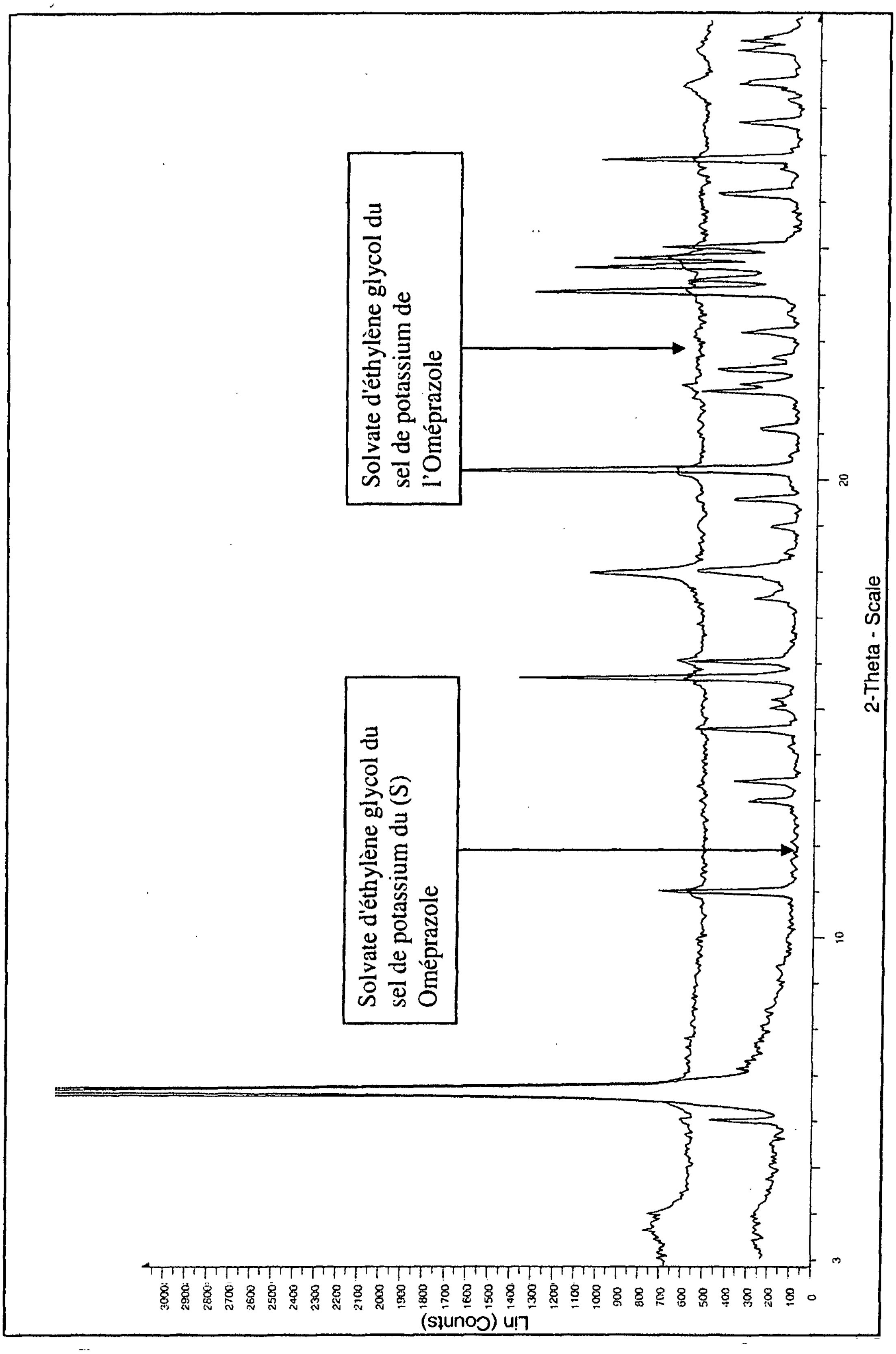


Figure 5 : Diffractogrammes de solvate d'éthylène glycol des sels de potassium de l'Oméprazole et du (S) Oméprazole

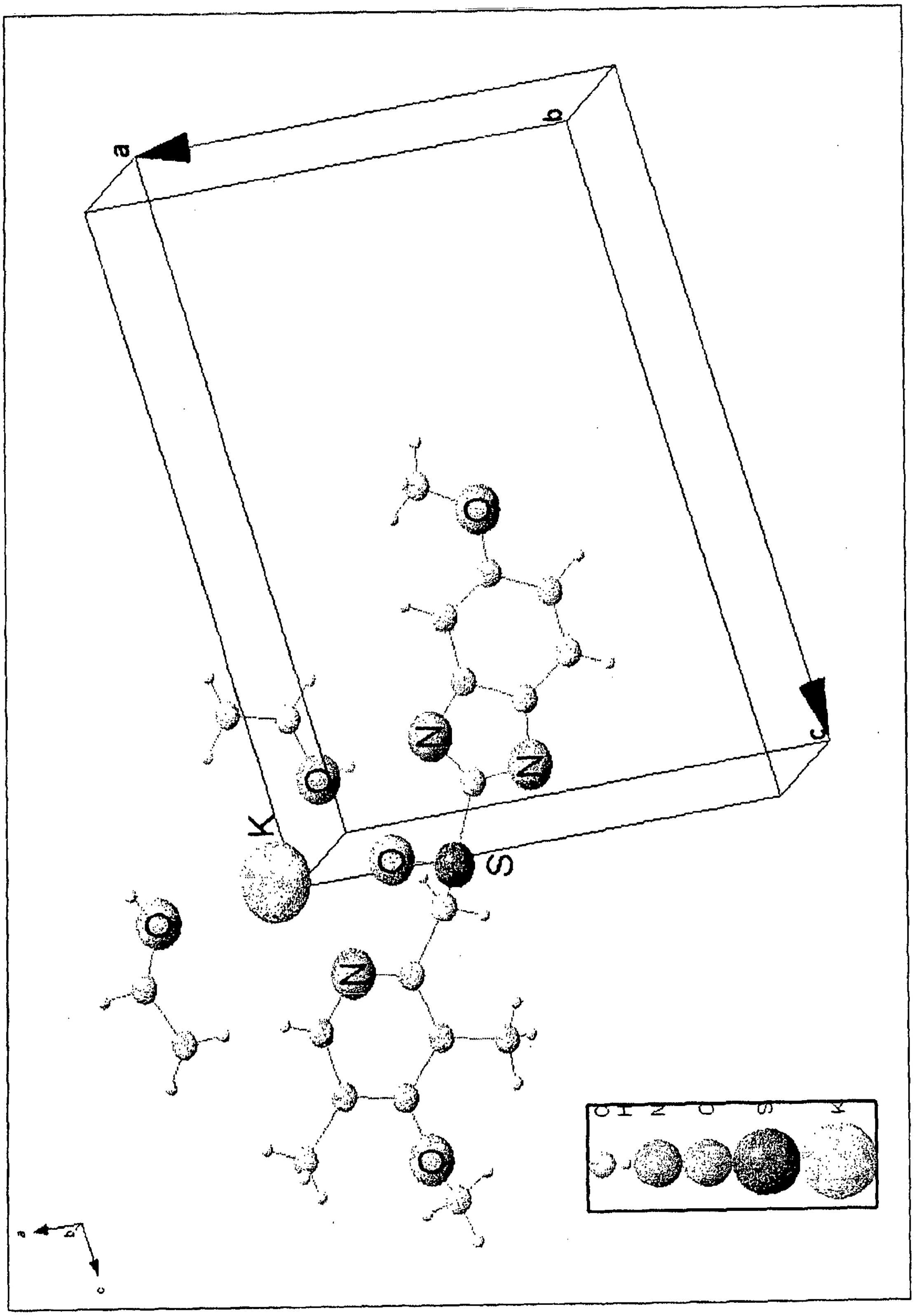

Figure 6 : Représentation 3D de l'unité asymétrique du solvate d'éthanol du sel de potassium du (S) Oméprazole

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0005129 A
- EP 124495 A
- US 4738974 A
- DE 4035455
- WO 9427988 A
- US 20060089386 A
- WO 9601623 A
- WO 9617077 A
- WO 9617076 A
- WO 9602535 A
- WO 2006040635 A
- WO 03089408 A
- WO 9828294 A
- WO 9854171 A
- WO 0044744 A
- WO 2004002982 A
- WO 2004046134 A
- WO 9702261 A
- EP 1498416 A
- WO 2004089935 A
- WO 2006001753 A
- WO 2006003163 A
- WO 2006134605 A
- WO 2004076440 A
- WO 2004020436 A
- WO 2007031845 A
- WO 2007049914 A
- WO 2007074099 A
- FR 2710337
- WO 9508522 A
- EP 0720595 A
- US 6022409 A
- WO 2004016569 A


### Littérature non-brevet citée dans la description


- *J. Chromatogr.,* 1990, vol. 532, 305-319
- *J. Phys. IV,* 2004, vol. 113, 11-15
- *Tetrahedron : Asymmetry,* 2000, vol. 11, 1729-1732
- An elusive concept ?. *Chem. Eng. Process,* 2006, vol. 45, 857-862
- Preferential Crystallization. **G. COQUEREL.** Topic in Current Chemistry, Novel Optical Resolution Technol ogi es. Springer, 2007, vol. 269, 1-51
- Preferential Crystallization. Topic in Current Chemistry, Novel Optical Resolution Technologies. Springer, 2007, vol. 269, 1-51
- **MADESCLAIRE M.** *Tetrahedron,* 1988, vol. 44, 6537-6580
- **ALLENMARK S.** *Acta. Chem. Scand.,* 1966, vol. 20, 910-911